# EUROPEAN PATENT APPLICATION

(11) **EP 3 949 993 A1**
(43) Date of publication of application: **09.02.2022**
(21) Application number: 20778102.2
(22) Date of filing: 30.03.2020
(51) Int. Cl.: A61K 45/00

(54) **CELL COMPETITION INHIBITOR**

(30) Priority: 28.03.2019 JP 2019063594; 01.04.2019 JP 2019069777
(71) Applicant: WASEDA UNIVERSITY, Shinjuku-ku Tokyo 169-8050 (JP)
(72) Inventor: MARUYAMA, Takeshi, Tokyo 169-8050 (JP)
(74) Representative: Heubeck, Christian
(86) International application number: PCT/JP2020/014715
(87) International publication number: WO 2020/196926

(57) **Abstract**

A cell competition regulator containing a protein containing α3 domain of MHC class Ia or an agonist antibody against LILRB3, or a substance that inhibits binding between MHC class Ia and LILRB3 is provided.

## Description

### [Technical Field]

The present invention relates to a novel regulator of cell competition.

### [Background Art]

Cell competition was originally discovered in Drosophila in 1975 (non-patent document 1), and has been studied by Drosophila researchers. Until recently, it was not clear whether similar competitive phenomenon exists in mammalian cells. In 2009, the group of the present inventors reported for the first time in the world that cell competition also occurs in mammals (non-patent document 2), and it has now been clarified that cell competition is a universal phenomenon that is necessary for maintaining homeostasis of cell society, and occurs in various physiological and pathological processes. In addition, recent research has gradually accumulated findings as to the molecular mechanism of how cells that have become loser in cell competition are eliminated from tissues (non-patent document 3).

### [Document List]

### [Non-patent documents]

non-patent document 1: Morata, G. et al., Dev. Biol., 1975; 42: 211-221
non-patent document 2: Hogan, C. et al., Nat Cell Biol. 2009 Apr; 11 (4) :460-7
non-patent document 3: Maruyama T. et al., Curr Opin Cell Biol. 2017; 48: 106-112

### [Summary of Invention]

### [Technical Problem]

However, the intercellular recognition mechanism called "the mechanism that induces the ability to eliminate epithelial cells by antigen presentation" and the intercellular communication molecule have been completely unknown to date. Therefore, the problem of the present invention is to elucidate the molecular entity responsible for the "mechanism that induces the ability to eliminate epithelial cells by antigen presentation" which entity is involved in cell competition in mammals, and to provide a cell competition regulator utilizing the mechanism, a method for regulating cell competition by using the regulator, and the like.

As mentioned above, 80% or more of the cancers that occur in our body are derived from epithelial cells. The present inventors had an idea that preventive medicine targeting the treatment of ultra-early cancer to "remove mutant cells before carcinogenesis" can be developed by applying the "mechanism that induces the ability to eliminate epithelial cells by antigen presentation" to medicine, or the above-mentioned mechanism may be applicable to the treatment of cancer by removing cancer cells by utilizing the above-mentioned mechanism even in the case of advanced stage cancer. To elucidate the above-mentioned mechanism, the present inventor first took note of the oncogene RasV12, which is known to promote cell competition when introduced into epithelial cells. When the gene was introduced into a human skin-derived cell line and RasV12 was expressed in the cells, the cell membrane expression of major histocompatibility complex (hereinafter to be referred to as "MHC") class I was promoted. Based on this finding, the present inventor considered that MHC class I and cell competition may be involved, and expressed RasV12 in TAP1-deficient cells known to suppress expression of MHC-I. As a result, it was shown that cell competition is not promoted, that is, cell membrane expression of MHC class I is necessary for cell competition. Then focusing on canine MHC-I DLA, individual DLA was deleted from the strain introduced with RasV12. As a result, it was shown that MHC-Ia DLA88 plays an essential role in cell competition. Then, the full-length peptide of DLA88 was administered to cells, but it does not promote cell competition. However, administration of a peptide fragment consisting of α3 domain among the three domains constituting the extracellular domain to cells surprisingly promotes cell competition. That is, it was newly found that the α3 domain of MHC-Ia is important for cell competition.

From the above-mentioned finding, it was assumed that the interaction between MHC class I and its receptor may be involved in cell competition, and attempts were made to identify the receptor for MHC class I. As a result, it was found that immunoglobulin-like receptor LILRB3 (leukocyte immunoglobulin-like receptor, subfamily B, member 3) functions as a receptor for MHC class I. Therefore, they focused on LILRB3 and proceeded with the research. As a result, they have found that
(i) knockout of the immunoglobulin-like receptor LILRB3 (leukocyte immunoglobulin-like receptor, subfamily B, member 3) on the epithelial cell membrane reduces the ability to eliminate abnormal cells,
(ii) recombinant protein containing the D1 - D2 domain among the four immunoglobulin domains D1 - D4 of LILRB3 binds to a recombinant protein containing the α3 domain of MHC class 1a, and further, (iii) a recombinant protein containing the D1-D2 domain of LILRB3 shows a suppressive effect on abnormal cells due to cell competition. Based on these findings, they have conducted further studies and completed the present invention.

### [Solution to Problem]

Accordingly, the present invention relates to the following.
[1] A cell competition regulator comprising a protein containing α3 domain of MHC class Ia or an agonist antibody against LILRB3, or a substance that inhibits binding between MHC class Ia and LILRB3.
[2] The regulator of [1], wherein the regulator comprises the protein containing α3 domain of MHC class Ia and the cell competition is regulated by promoting cell competition.
[3] The regulator of [2], wherein the MHC class Ia is HLA-B or DLA-88.
[4] The regulator of [2] or [3], wherein the protein containing α3 domain of MHC class Ia comprises at least one protein selected from the group consisting of the following:
   a) a protein having the amino acid sequence shown in SEQ ID NO: 1,
   b) a protein having an amino acid sequence resulting from deletion, substitution, or addition of one to several amino acids in the amino acid sequence shown in SEQ ID NO: 1, and specifically binding to a protein containing D1 and D2 domains of LILRB3,
   c) a protein having an amino acid sequence having at least 90% similarity to the amino acid sequence shown in SEQ ID NO: 1, and specifically binding to a protein containing D1 and D2 domains of LILRB3, and
   d) a protein containing α3 domain of an orthologue of DLA-88.
[5] The regulator of [4], wherein the protein containing the D1 and D2 domains of LILRB3 is a protein having the amino acid sequence shown in SEQ ID NO: 8.
[6] The regulator of any one of [2] to [5], wherein the cell competition is regulated by eliminating abnormal cells by a normal cell not belonging to an immune system.
[7] The regulator of [6], wherein the normal cell not belonging to an immune system is an epithelial cell.
[8] The regulator of [6] or [7], wherein the abnormal cell is a mutant cell, a cancer cell, or a virus-infected cell.
[9] The regulator of [8], wherein the regulator is a prophylactic or therapeutic agent for cancer or a virus infectious disease.
[10] The regulator of [1], wherein the regulator comprises the substance that inhibits binding between MHC class Ia and LILRB3 and the cell competition is regulated by suppressing cell competition.
[11] The regulator of [10], wherein the substance that inhibits binding between MHC class Ia and LILRB3 is at least one kind of substance selected from the group consisting of a protein containing D1 and D2 domains of LILRB3, an antibody against D1 and D2 domains of LILRB3, an aptamer, and an inhibitor of LILRB3 expression.
[12] The regulator of [11], wherein the protein containing D1 and D2 domains of LILRB3 comprises at least one protein selected from:
   e) a protein having the amino acid sequence shown in SEQ ID NO: 8,
   f) a protein having an amino acid sequence resulting from deletion, substitution, or addition of one to several amino acids in the amino acid sequence shown in SEQ ID NO: 8, and specifically binding to a protein containing α3 domain of MHC class Ia,
   g) a protein having an amino acid sequence having at least 80% similarity to the amino acid sequence shown in SEQ ID NO: 8, and specifically binding to a protein containing α3 domain of MHC class Ia, and
   h) a protein containing D1 and D2 domains of an orthologue of ENSCAFG00000028453.
[13] The regulator of any one of [10] to [12], wherein the cell competition is regulated by eliminating transplanted cells by a normal cell not belonging to an immune system.
[14] The regulator of any one of [10] to [12], wherein the cell competition is suppressed by improving an engraftment failure in organ transplantation, tissue transplantation, or cell transplantation, or suppressing progression of symptoms of a neurodegenerative disease, a muscular degenerative disease, or other various denaturation diseases.
[15] The regulator of [14], wherein the organ transplantation is transplantation of at least one kind of organ selected from the group consisting of liver, kidney, heart, lung, pancreas, thyroid gland, parathyroid, thymus, an endocrine organ including adrenal cortex and adrenal medulla, and an organ containing stem cells.
[16] The regulator of [14], wherein the tissue transplantation is transplantation of at least one kind of tissue selected from the group consisting of skin, a tissue containing stem cells, and a tissue containing immunocytes.
[17] The regulator of [14], wherein the cell transplantation is transplantation of at least one kind of cell selected from the group consisting of bone marrow cell, non-adherent bone marrow cell, peripheral blood cell, cord blood cell, Wharton jelly-derived cell, placenta-derived cell, hair root-derived cell, adipose tissue-derived cell, lymphocyte, monocyte and macrophage.
[18] The regulator of [14], wherein the various denaturation diseases are cervical spondylotic myelopathy and/or lumbar canal stenosis.
[19] The regulator of [14], wherein the neurodegenerative disease is at least one kind of neurodegenerative disease selected from the group consisting of Alzheimer's disease, mild cognitive impairment, Parkinson's disease, Huntington's disease, spinocerebellar ataxia, Creutzfeldt-Jakob disease, trauma-induced neurodegeneration, high-pressure nervous syndrome, dystonia, olivopontoserebellar atrophy, amyotrophic lateral sclerosis, multiple sclerosis, epilepsy, dementia, senile dementia, AIDS-dementia composite, and AIDS-induced encephalopathy.
[20] The regulator of [14], wherein the muscular degenerative disease is at least one kind of muscular degenerative disease selected from the group consisting of muscular dystrophy, distal myopathy, congenital myopathy, thyrotoxic myopathy, glycogen storage disease, mitochondrial myopathy, steroid myopathy, alcoholic myopathy, inflammatory myopathy, endocrine myopathy, lipid storage disease myopathy, myopathy associated with infections such as HIV, vitreous body myopathy, and myopathy associated with an autoimmune disease such as myasthenia gravis.
[21] The regulator of [14], which is an agent for improving an engraftment failure in organ transplantation, tissue transplantation, or cell transplantation, or an agent for suppressing progression of symptoms of a neurodegenerative disease, a muscular degenerative disease, or other various denaturation diseases.
[22] The regulator of any of [1] to [21], which is used in combination with other medicament and/or other treatment method.
[23] The regulator of [22], wherein said other medicament is an anticancer agent or an antiviral agent.
[24] The regulator of [23], wherein the anticancer agent is at least one kind of anticancer agent selected from the group consisting of alkylating agent, cytotoxic antibiotic, platinum preparation, antimetabolite, kinase inhibitor, angiogenesis inhibitor, hormonal agent, DNA modifying enzyme inhibitor, proteosome inhibitor, alkaloid agent, type I and type II topoisomerase inhibitors, histone deacetylase inhibitor, cytokine preparation, hormonal agent, immune checkpoint inhibitor, natural killer cell activator, indoleamine 2,3-dioxygenase (IDO) inhibitor, monoclonal antibody, and other molecular-targeted therapeutic agent.
[25] The regulator of [23], wherein the antiviral agent is at least one kind of antiviral agent selected from the group consisting of interferon, nucleosidic and nucleotidic reverse transcriptase inhibitor, non-nucleosidic reverse transcriptase inhibitor, protease inhibitor, integrase inhibitor, fusion inhibitor, mature inhibitor, guanosine analog, pridin analog, pyrimidine analog, and other "unclassified" antiviral agent recognized in the art and is not in any of the above-mentioned classes (e.g., foscarnet and miltefosine).
[26] The regulator of [22], wherein the other treatment method is at least one kind of method selected from the group consisting of cancer surgery, radiation therapy, laser radiation therapy, and hyperthermic therapy.
[27] The regulator of [22], wherein the other medicament is at least one kind of medicament selected from the group consisting of an immunosuppressant, a rejection inhibitor for transplanted organ, and a post-transplantation engraftment promoter.
[28] The regulator of [27], wherein the immunosuppressant is at least one kind of immunosuppressant selected from the group consisting of steroid, cyclosporine, cyclosporine analogue, cyclophosphamide, methylprednisone, prednisone, azathioprine, tacrolimus hydrate, 15-deoxyspergualin, natalizumab, rapamycin, and etanercept.
[29] The regulator of any one of [1] to [28], wherein the regulator is a pharmaceutical composition.
[30] A method for regulating cell competition, comprising administering a therapeutically effective amount of a protein containing α3 domain of MHC class Ia or an agonist antibody against LILRB3, or a substance that inhibits binding between MHC class Ia and LILRB3 to a subject in need thereof.
[31] The method of [30], wherein the protein containing α3 domain of MHC class Ia or an agonist antibody against LILRB3 is administered, and the cell competition is regulated by promoting cell competition.
[32] The method of [31], wherein the MHC class Ia is HLA-B or DLA-88.
[33] The method of [31] or [32], wherein the protein containing α3 domain of MHC class Ia comprises at least one protein selected from the group consisting of the following:
   a) a protein having the amino acid sequence shown in SEQ ID NO: 1,
   b) a protein having an amino acid sequence resulting from deletion, substitution, or addition of one to several amino acids in the amino acid sequence shown in SEQ ID NO: 1, and specifically binding to a protein containing D1 and D2 domains of LILRB3,
   c) a protein having an amino acid sequence having at least 90% similarity to the amino acid sequence shown in SEQ ID NO: 1, and specifically binding to a protein containing D1 and D2 domains of LILRB3, and
   d) a protein containing α3 domain of an orthologue of DLA-88.
[34] The method of [33], wherein the protein containing the D1 and D2 domains of LILRB3 is a protein having the amino acid sequence shown in SEQ ID NO: 8.
[35] The method of any one of [31] to [34], wherein the cell competition is regulated by eliminating abnormal cells by normal cell not belonging to an immune system.
[36] The method of [35], wherein the normal cell not belonging to an immune system is an epithelial cell.
[37] The method of [35] or [36], wherein the abnormal cell is a mutant cell, a cancer cell, or a virus-infected cell.
[38] The method of [37], wherein the cell competition is regulated by preventing or treating cancer or a virus infectious disease.
[39] The method of [30], wherein the substance that inhibits binding between MHC class Ia and LILRB3 is administered, and the cell competition is regulated by suppressing cell competition.
[40] The method of [39], wherein the substance that inhibits binding between MHC class Ia and LILRB3 is at least one kind of substance selected from the group consisting of a protein containing D1 and D2 domains of LILRB3, an antibody against D1 and D2 domains of LILRB3, an aptamer, and an inhibitor of LILRB3 expression.
[41] The method of [39] or [40], wherein the protein containing D1 and D2 domains of LILRB3 comprises at least one protein selected from the following:
   e) a protein having the amino acid sequence shown in SEQ ID NO: 8,
   f) a protein having an amino acid sequence resulting from deletion, substitution, or addition of one to several amino acids in the amino acid sequence shown in SEQ ID NO: 8, and specifically binding to a protein containing α3 domain of MHC class Ia,
   g) a protein having an amino acid sequence having at least 80% similarity to the amino acid sequence shown in SEQ ID NO: 8, and specifically binding to a protein containing α3 domain of MHC class Ia, and
   h) a protein containing D1 and D2 domains of an orthologue of ENSCAFG00000028453.
[42] The method of any one of [39] to [41], wherein the cell competition is regulated by eliminating transplanted cells by a normal cell not belonging to an immune system.
[43] The method of any one of [39] to [42], wherein the cell competition is suppressed by improving an engraftment failure in organ transplantation, tissue transplantation, or cell transplantation, or suppressing progression of symptoms of a neurodegenerative disease, a muscular degenerative disease, or other various denaturation diseases.
[44] The method of [43], wherein the organ transplantation is transplantation of at least one kind of organ selected from the group consisting of liver, kidney, heart, lung, pancreas, thyroid gland parathyroid, thymus, an endocrine organ including adrenal cortex and adrenal medulla, and an organ containing stem cells.
[45] The method of [43], wherein the tissue transplantation is transplantation of at least one kind of tissue selected from the group consisting of skin, tissue containing stem cells, and tissue containing immunocytes.
[46] The method of [43], wherein the cell transplantation is transplantation of at least one kind of cell selected from the group consisting of bone marrow cell, non-adherent bone marrow cell, peripheral blood cell, cord blood cell, Wharton jelly-derived cell, placenta-derived cell, hair root-derived cell, adipose tissue-derived cell, lymphocyte, monocyte, and macrophage.
[47] The method of [43], wherein the various denaturation diseases are cervical spondylotic myelopathy and/or lumbar canal stenosis.
[48] The method of [43], wherein the neurodegenerative disease is at least one kind of neurodegenerative disease selected from the group consisting of Alzheimer's disease, mild cognitive impairment, Parkinson's disease, Huntington's disease, spinocerebellar ataxia, Creutzfeldt-Jakob disease, trauma-induced neurodegeneration, high-pressure nervous syndrome, dystonia, olivopontoserebellar atrophy, amyotrophic lateral sclerosis, multiple sclerosis, epilepsy, dementia, senile dementia, AIDS-dementia composite, and AIDS-induced encephalopathy.
[49] The method of [43], wherein the muscular degenerative disease is at least one kind of muscular degenerative disease selected from the group consisting of muscular dystrophy, distal myopathy, congenital myopathy, thyrotoxic myopathy, glycogen storage disease, mitochondrial myopathy, steroid myopathy, alcoholic myopathy, inflammatory myopathy, endocrine myopathy, lipid storage disease myopathy, myopathy associated with infections such as HIV, vitreous body myopathy, and myopathy associated with an autoimmune disease such as myasthenia gravis.
[50] The method of any of [30] to [49], which is used in combination with administration of other medicament and/or other treatment method.
[51] The method of [50], wherein said other medicament is an anticancer agent or an antiviral agent.
[52] The method of [51], wherein the anticancer agent is at least one kind of anticancer agent selected from the group consisting of alkylating agent, cytotoxic antibiotic, platinum preparation, antimetabolite, kinase inhibitor, angiogenesis inhibitor, hormonal agent, DNA modifying enzyme inhibitor, proteosome inhibitor, alkaloid agent, type I and type II topoisomerase inhibitors, histone deacetylase inhibitor, cytokine preparation, hormonal agent, immune checkpoint inhibitor, natural killer cell activator, indoleamine 2,3-dioxygenase(IDO) inhibitor, monoclonal antibody, and other molecular-targeted therapeutic agent.
[53] The method of [51], wherein the antiviral agent is at least one kind of antiviral agent selected from the group consisting of interferon, nucleoside and nucleotide reverse transcriptase inhibitor, non-nucleoside reverse transcriptase inhibitor, protease inhibitor, integrase inhibitor, fusion inhibitor, mature inhibitor, guanosine analog, pridin analog, pyrimidine analog, and other "unclassified" antiviral agent recognized in the art and is not in any of the above-mentioned classes (e.g., foscarnet and miltefosine).
[54] The method of [50], wherein the other treatment method is at least one kind of method selected from the group consisting of cancer surgery, radiation therapy, laser radiation therapy, and hyperthermic therapy.
[55] The method of [50], wherein the other medicament is at least one kind of medicament selected from the group consisting of an immunosuppressant, a rejection inhibitor for transplanted organ, and a post-transplantation engraftment promoter.
[56] The method of [55], wherein the immunosuppressant is at least one kind of immunosuppressant selected from the group consisting of steroid, cyclosporine, cyclosporine analogue, cyclophosphamide, methylprednisone, prednisone, azathioprine, tacrolimus hydrate, 15-deoxyspergualin, natalizumab, rapamycin, and etanercept.
[57] A protein comprising α3 domain of MHC class Ia, or a protein comprising D1 and D2 domains of LILRB3 for use in regulating cell competition.
[58] The protein of [57], which is the protein containing α3 domain of MHC class Ia, wherein the cell competition is regulated by promoting cell competition.
[59] The protein of [58], wherein the MHC class Ia is HLA-B or DLA-88.
[60] The protein of [57], which is the protein containing D1 and D2 domains of LILRB3, wherein the cell competition is regulated by suppressing cell competition.
[61] Use of a protein containing α3 domain of MHC class Ia or an agonist antibody against LILRB3, or a substance that inhibits binding between MHC class Ia and LILRB3 in the production of a medicament for regulating cell competition.
[62] The use of [61], wherein the protein containing α3 domain of MHC class Ia or the agonist antibody against LILRB3 is used, and the cell competition is regulated by promoting cell competition.
[63] The protein of [60], wherein the MHC class Ia is HLA-B or DLA-88.
[64] The use of the protein of [61], wherein the substance that inhibits binding between MHC class Ia and LILRB3 is used, and the cell competition is regulated by suppressing cell competition.
[65] The method of [64], wherein the substance that inhibits binding between MHC class Ia and LILRB3 is at least one kind of substance selected from the group consisting of a protein containing D1 and D2 domains of LILRB3, an antibody against D1 and D2 domains of LILRB3, an aptamer, and an inhibitor of LILRB3 expression.

### [Advantageous Effects of Invention]

According to the present invention, a cell competition regulator, a method for regulating cell competition, a protein for use in a method for regulating cell competition, use of a protein for producing a medicament for regulating cell competition, and the like can be provided. The regulator of the present invention regulates cell competition by regulating the interaction between MHC class Ia and LILRB3 which is a receptor therefor, which in turn makes it possible to prevent or treat neurodegenerative diseases (e.g., amyotrophic lateral sclerosis, Alzheimer's disease, etc.), muscular degenerative diseases (e.g., muscular dystrophy), cancer (also including precancer) (e.g., pancreatic cancer, lung cancer, skin cancer, uterine cancer, etc.), bacterium infection or virus infection (e.g., human papillomavirus (HPV), etc.), cell transplant engraftment failure (e.g., induced pluripotent stem cell (iPS cell) transplantation, cardiomyocyte transplantation, cornea transplantation, skin transplantation, etc.).

### [Brief Description of Drawings]

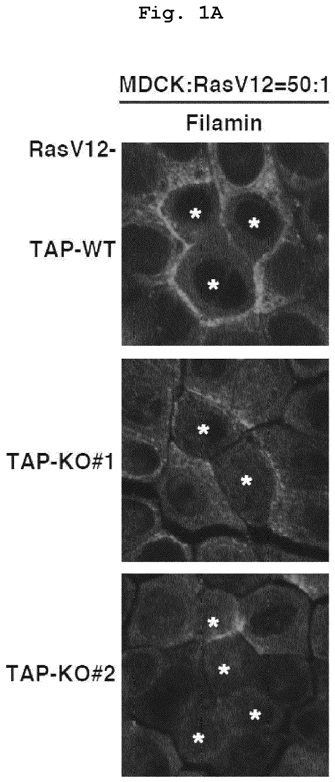

Fig. 1A shows micrographs of a fluorescence microscope observing the accumulation of Filamin by normal cells, which examines whether TAP1-deficient mutation in RasV12-expressing cells promotes elimination by normal cells (MDCK). The upper panel (TAP-WT): when RasV12-expressing cell having wild-type TAP1 was used. Middle panel (TAP1-KO#1) and lower panel (TAP1-KO#2): when TAP1-deficient RasV12-expressing cell was used. * shows RasV12-expressing cell.

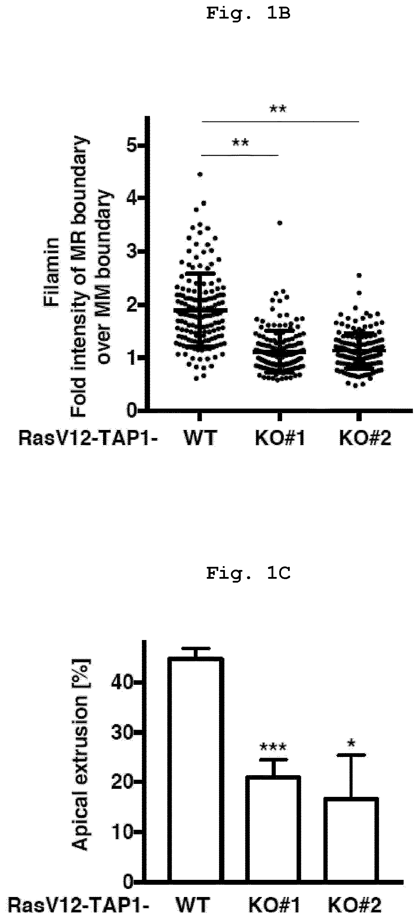

Fig. 1B shows measurement and comparison of Filamin accumulation. The data shows mean±SD (n=3). **:p<0.01 (Student's t-test) .
Fig. 1C shows proportions of apical extrusiion which indicates cell elimination ability. The data shows mean±SD (n=3). *:p<0.05, ***:p<0.001 (Student's t-test) .

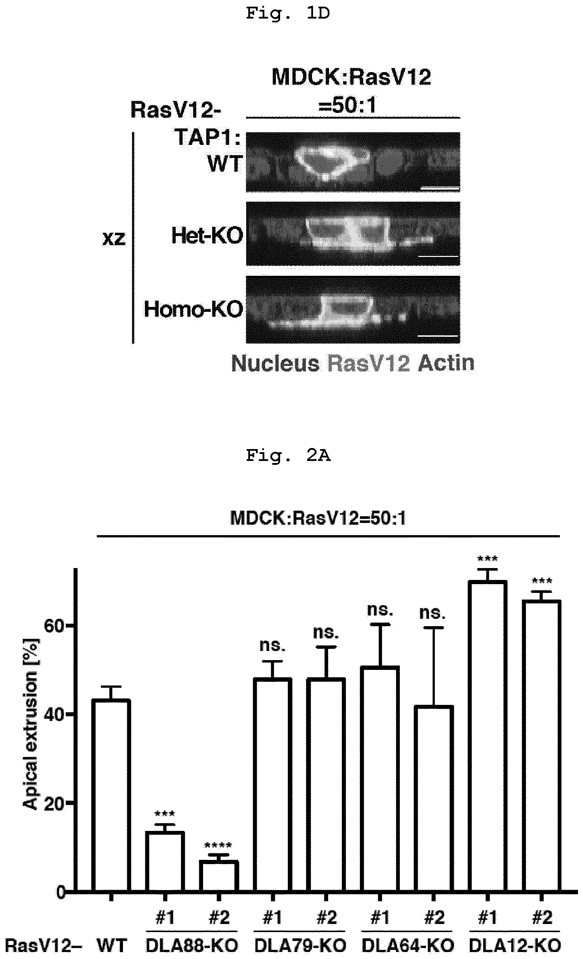

Fig. 1D shows representative XZ images of normal and RasV12 cells when MDCK-RasV12-WT or -TAP^{KO} cells were mixed with normal MDCK cells on a collagen gel and, after monolayer formation, treated with doxycycline for 24 hr, and the cells were fixed and stained with phalloidin and Hoechst. Scale bar: 10 µm
Fig. 2A shows the evaluation results of the effect of normal cells (MDCK) on the elimination of RasV12-expressing cells when various DLA families are deleted from RasV12-expressing cells. ***:p<0.001, ****:p<0.0001, ns.: no significant difference (Student's t-test).

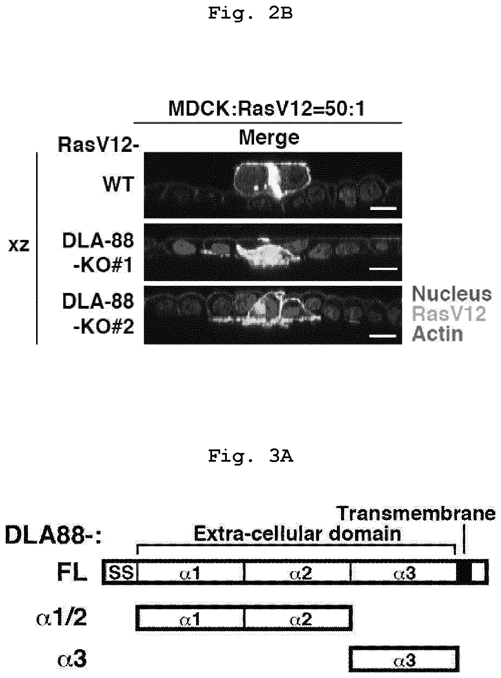

Fig. 2B shows images of the behavior of RasV12-expressing cells analyzed with a confocal microscope.
Fig. 3A is a schematic showing of the structures of full-length protein (FL) of DLA-88, protein containing only α1 and α2 domains of DLA88 (DLA88-α1/2), and protein containing only α3 domain of DLA88 (DLA88-α3).

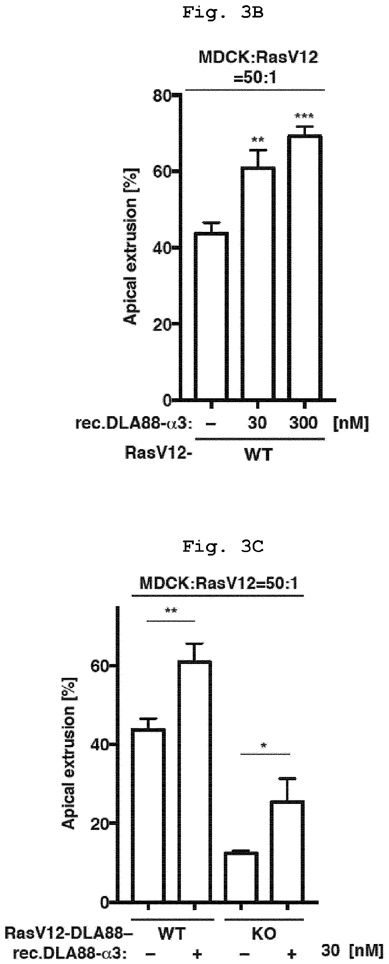

Fig. 3B shows observation results of the elimination of the RasV12-expressing cell when the cells were blended at a ratio of MDCK normal cells:RasV12 expression MDCK cells=50:1, a single cell layer was formed, doxycycline and recombinant protein DLA88-α3 were simultaneously added and the cells were treated. **:p<0.01, ***:p<0.01 (Student's t-test).
Fig. 3C shows observation results of the elimination of the DLA88 deficient RasV12-expressing cells when the cells were blended at a ratio of normal cells:DLA88 deficient RasV12-expressing cells=50:1, doxycycline and recombinant protein DLA88-α3 were simultaneously added and the cells were treated. *:p<0.05, **:p<0.01 (Student's t-test).

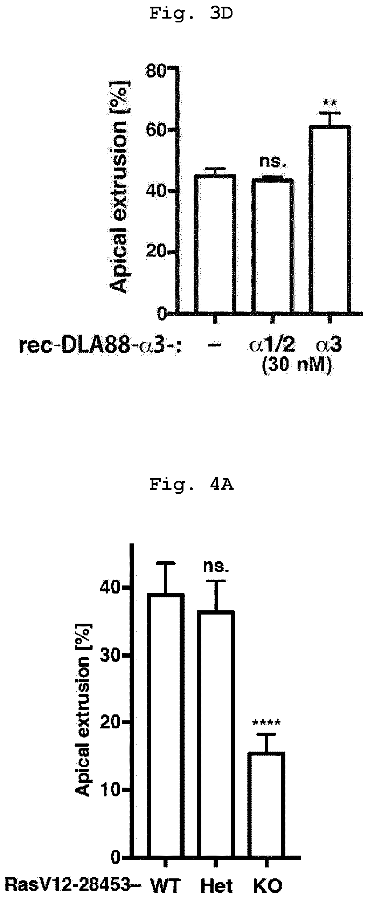

Fig. 3D shows observation results of the elimination of the RasV12-expressing cell when the cells were blended at a ratio of MDCK normal cells:RasV12 expression MDCK cells=50:1, a single cell layer was formed, doxycycline alone, doxycycline and recombinant protein DLA88-α1/2, or doxycycline and recombinant protein DLA88-α3 were simultaneously added and the cells were treated. **:p<0.01 (Student's t-test).
Fig. 4A shows the efficiency of elimination of RasV12-expressing cells deficient in the gene of the cell membrane protein LILRB3 (ENACAFG000028453) that binds to MHC class I.

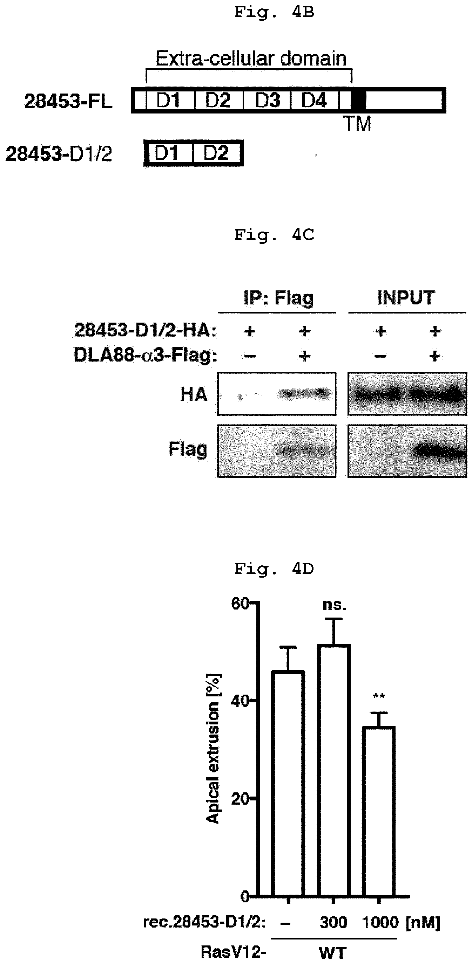

Fig. 4B is a schematic showing of the structures of the full-length protein (28453-FL) of LILRB3 (ENACAFG000028453) and the recombinant protein (28453-D1/2) consisting only of the D1 and D2 domains which was used in the experiment.
Fig. 4C is a blot drawing showing that a recombinant protein containing D1/D2 domain of LILRB3 (28453-D1/2) and a recombinant protein containing DLA88-α3 domain (DLA88-α3) bind to each other in vitro.
Fig. 4D shows evaluation results of the effect of addition of a recombinant protein containing a recombinant protein (28453-D1/2) of the D1/D2 domain of LILRB3 on the elimination effect of normal MDCK cells on RasV12-expressing MDCK.

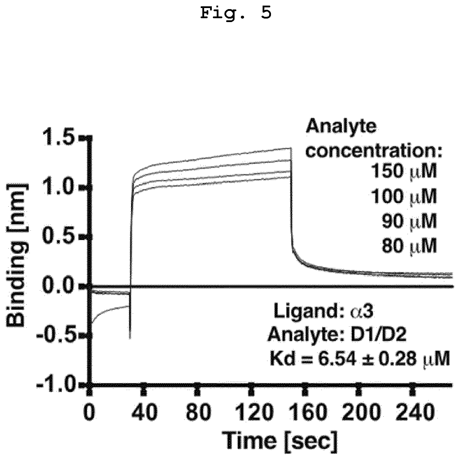

Fig. 5 shows kinetic analysis between DLA88 and LILRB3 by using a biolayer interference method. The recombinant proteins DLA88-α3 and LILRB3-D1/D2 used in (b) were separately incubated at 37°C for 2 hr, and immobilized on the BLI sensor for 5 min using DLA88-α3 as a ligand. LILRB3-D1/2D was loaded as a sample at the indicated concentrations. Kd = 6.54±0.28 µM.

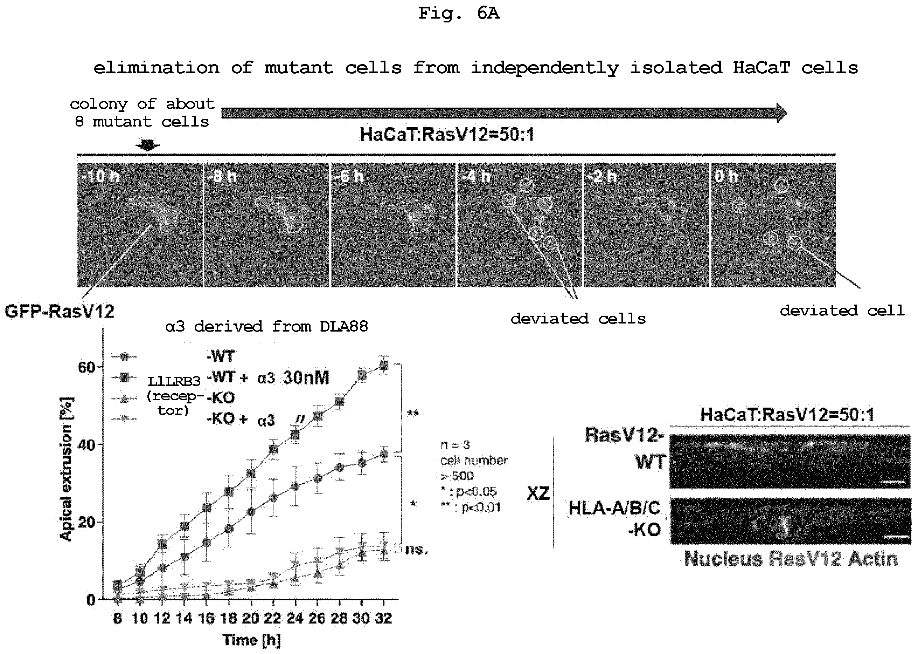

Fig. 6A Upper figure shows elimination of mutant cells from independently isolated HaCaT cells. Lower left figure: HaCaT-RasV12 cells were mixed with HaCaT-WT or HaCaT-LILRB3KO and treated with doxycycline with or without α3. Live imaging analysis was performed from 8 hr to 56 hr at a rate of one image every 5 min. Extruded cells were counted at the indicated time and the efficiency of apical extrusion is shown in a graph. Under the present experimental conditions, n≥538 cells. The data shows mean±SEM from 3 independent experiments. * P <0.05, ** P <0.01 (Student's one way anova). Lower right figure: Knockout of HLA in RasV12 cells made the efficiency of apical extrusion disappear. HaCaT-RasV12-WT or -HLA tripleknockout (HLAtKO) cells were mixed with normal HaCaT cells on a collagen gel and, after monolayer formation, treated with doxycycline for 16 hr, and the cells were fixed and stained with phalloidin and Hoechst. Representative XZ images of normal cells and RasV12 cells. Scale bar: 10 µm.

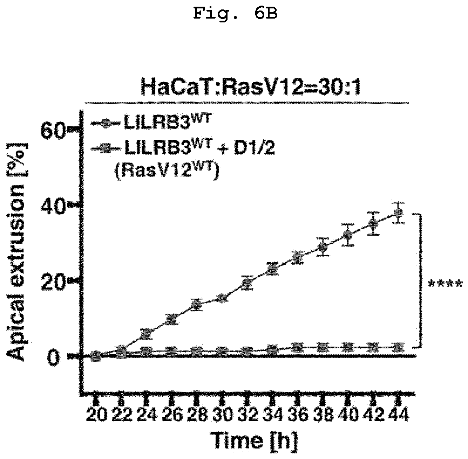

Fig. 6B shows observation results of elimination of RasV12-expressing HaCaT cells by normal HaCaT cells under conditions of normal human HaCaT cells:RasV12-expressing human HaCat cells without LILRB3WT gene deficiency (LILRB3^{WT}-RasV12) = 30:1, when doxycycline and recombinant protein 28453-D1/2 were simultaneously added for treatment (LILRB3^{WT}+28453-D1/2) and when doxycycline alone was added for treatment (LILRB3^{WT}). ****:p<0.01.

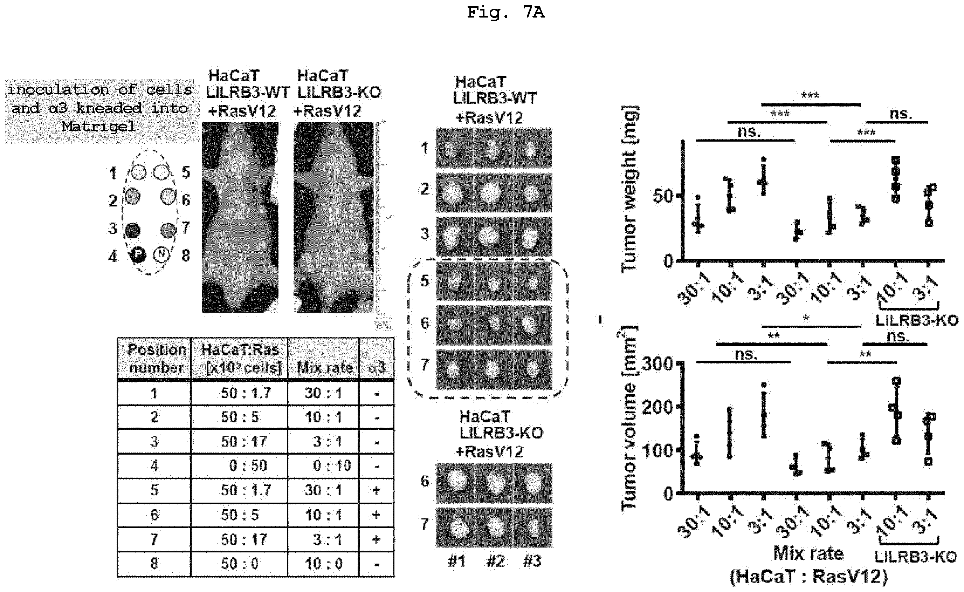

Fig. 7A Upper left figure: positions of subcutaneous injection. Lower left figure: injection conditions. Center figure: tumor image. Tumor was placed on a board divided into 1 cm × 1 cm squares. Right figure: measurement results of the total weight or total volume of tumor clump formed in 2 weeks. ns.: no significant difference, *:p<0.05, **:p<0.01 and ***:p<0.005 (Student's t-test).

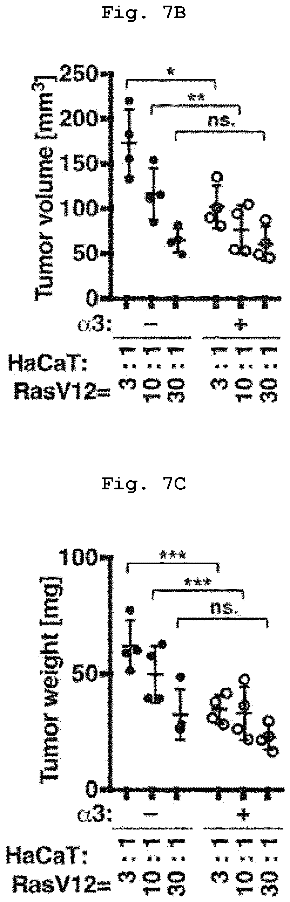

Fig. 7B shows measurement results of the total volume of tumor clump formed in 2 weeks from mixing human HaCat normal cells (HaCat):RasV12-expressing human HaCaT cells (RasV12) at 3:1, 10:1 or 30:1 with matrigel in the presence or absence of DLA88-α3, and transplantation of the pellet of the mixed cells to the abdomen of a nude mouse.
Fig. 7C shows measurement results of the total weight or total volume of tumor clump formed in 2 weeks from mixing human HaCat normal cells (HaCat):RasV12-expressing human HaCaT cells (RasV12) at 3:1, 10:1 or 30:1with matrigel and transplantation of the pellet of the mixed cells to the abdomen of a nude mouse. ns.: no significant difference, *:p<0.05, **:p<0.01 and ***:p<0.005 (Student's t-test).

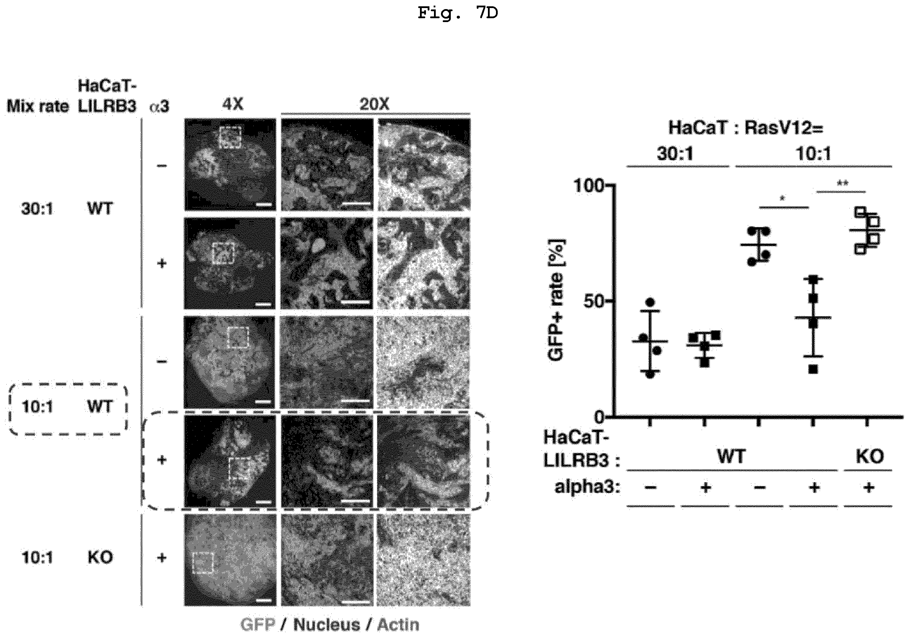

Fig. 7D Left figure: tumor tissue sections. Tumors were fixed and frozen sections were prepared. Sections were stained with phalloidin and Hoechst. Scale bar: 200 µm. Right figure: GFP positive rate in tumor sections. GFP-positive regions were quantified using image analysis software and the proportion of the regions was calculated using the whole region of each tumor. ns.: no significant difference, *:p<0.05 (Student's t-test).

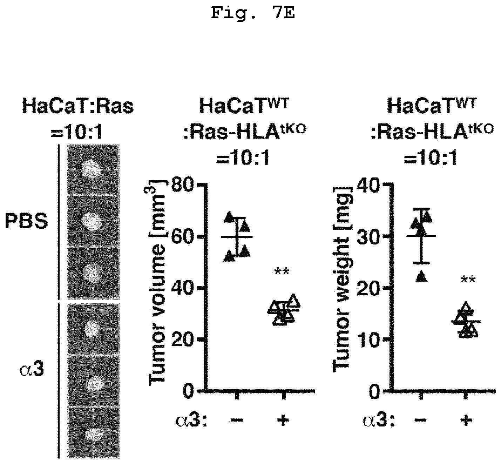

Fig. 7E Left figure: tumor images. Tumor was placed on a board divided into 1 cm × 1 cm squares. Center figure and Right figure: measurement results of the total weight or total volume of tumor clump formed in 2 weeks. **:p<0.01 and ***:p<0.005 (Student's t-test) .

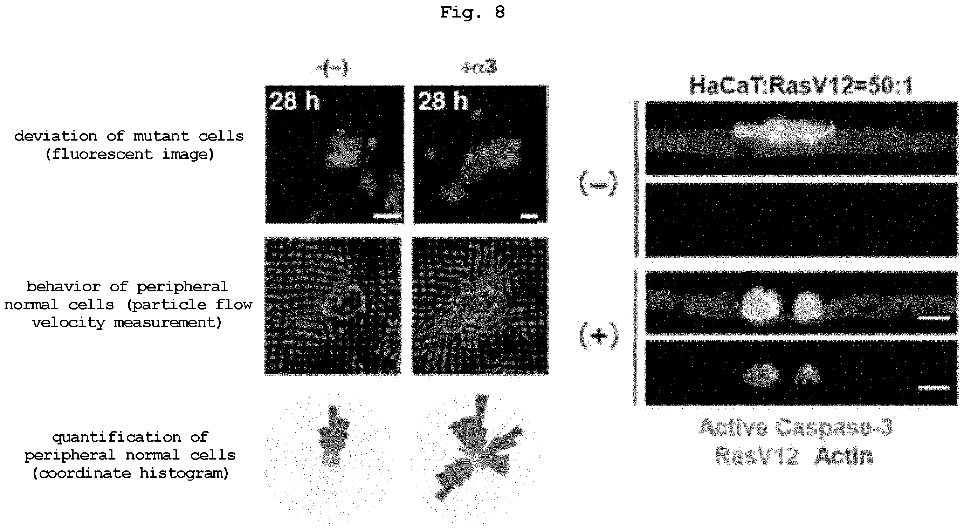

Fig. 8 Upper left figure: fluorescence images of HaCaT-RasV12 cells. Lower left figure: images of particle image velocimetry (PIV). Normal HaCaT and RasV12 cells were seeded on a thin collagen gel at a ratio of 30:1. After the cells formed a monolayer, the cells were cultured with doxycycline. After 8 hr of incubation, live imaging analysis was performed for 48 hr. PIV parameters were calculated from the movement of each cell. The arrows indicate the maximum (5 pixel/sec) and minimum (0 pixel/sec) mobility of the target cell. The video at 28 hr and the representative image of PIV are shown. Right figure: apoptosis is induced in the eliminated cells when the α3 domain is added (+).

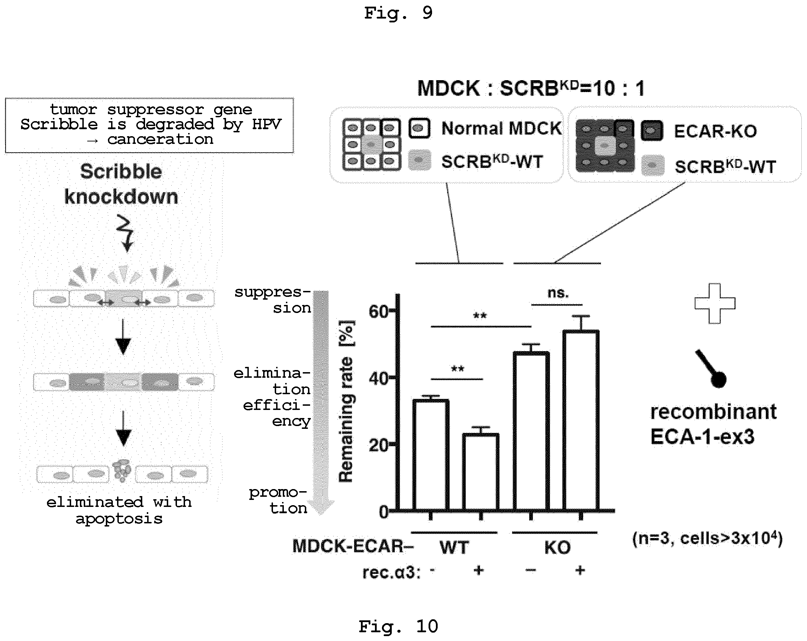

Fig. 9 Left figure: Human papillomavirus (HPV) is known to cause canceration by degrading the tumor suppressor gene Scribble after infecting cells. On the other hand, it is known that cells that suppress the expression of Scribble are eliminated by peripheral normal cells while inducing apoptosis. Right figure: elimination of Scribble knockdown cells is promoted via α3 (ECA-1-ex3) ECAR. Tetracycline-induced Scribble knockdown MDCK cells (GFP-positive cells) were mixed with normal cells or 28453 (ECAR) and seeded at a ratio of 1:10 (MDCK:SCRB^{KD} = 10:1). After forming a single phase, tetracycline and α3 were added, and the ratio of GFP-positive cells (Scribble knockdown cells) to the number of cells in the microscopic field 24 hr later was calculated. ns.: not significant, **P< 0.01.
Fig. 10 Elimination of HPV whole-genome stable cell MCF10A cells is promoted by α3 treatment.
   MCF10A (HPV18) cells that stably retain the whole HPV genome (HPV18 genome) were made distinguishable by CMFDA (green fluorescence staining) after mixed culture, mixed with normal MCF10A at the ratio of 10:1 (Normal MCF10A:HPV18 = 10:1), and then seeded. The ratio of GFP-positive cells (HPV18 cells) to the number of cells in the microscopic field 24 hr later was calculated. Cont: residual rate of HPV18 in epithelial cell layer, a3: residual rate of HPV18 cells upon α3 treatment. ns.: not significant, **P< 0.01.

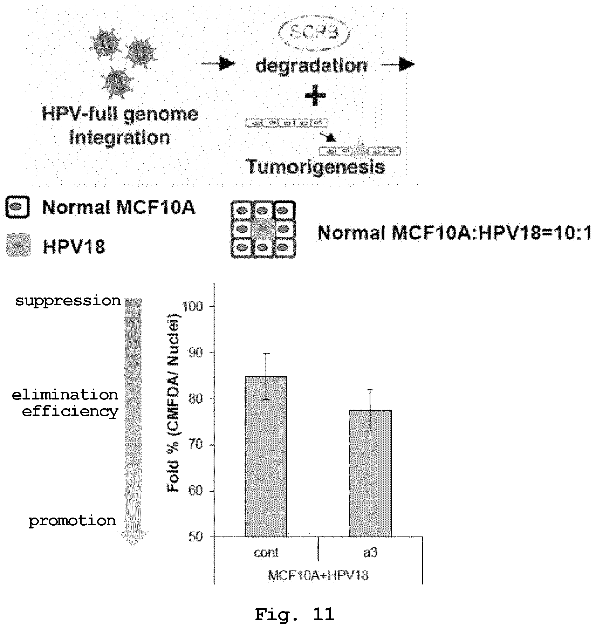

Fig. 11 Upper left figure: LILRB3 was up-regulated under the mixing conditions in quantitative PCR analysis. Normal MDCK or MDCK-RasV12 cells were seeded alone (single), or MDCK-RasV12 cells were mixed with normal MDCK cells at a ratio of 1:1 (mixing) on a silicone plate (elastic modulus: 0.5 kPa). After monolayer formation, the cells were treated with doxycycline for 8 hr. The cells were lysed and the sample was subjected to quantitative PCR analysis. Expression levels under the mixing conditions were compared with mean values under normal conditions and RasV12 alone conditions. The data shows mean±SD from 5 independent experiments. ** P <0.01 (Student's t-test). Lower left figure: Quantified data of LILRB3 induction. The data shows mean±SD of 3 independent experiments. Under each experiment condition, n=60 cells. * P <0.05 (Student's t-test). Right figure: Normal MDCK or MDCK-RasV12 cells alone (single) or MDCK-RasV12 cells were mixed with normal MDCK cells on a collagen gel. After 24 hr from doxycycline treatment, cells were fixed without permeation treatment and stained with anti-LILRB3 antibody and anti-HLA-B antibody. Representative XY images of normal cells and RasV12 cells. Scale bar: 10 µm.

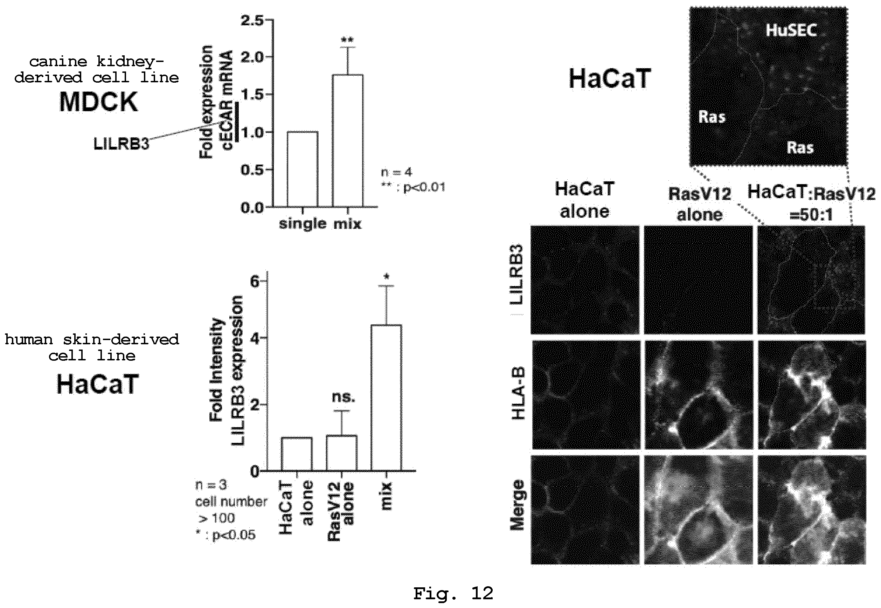

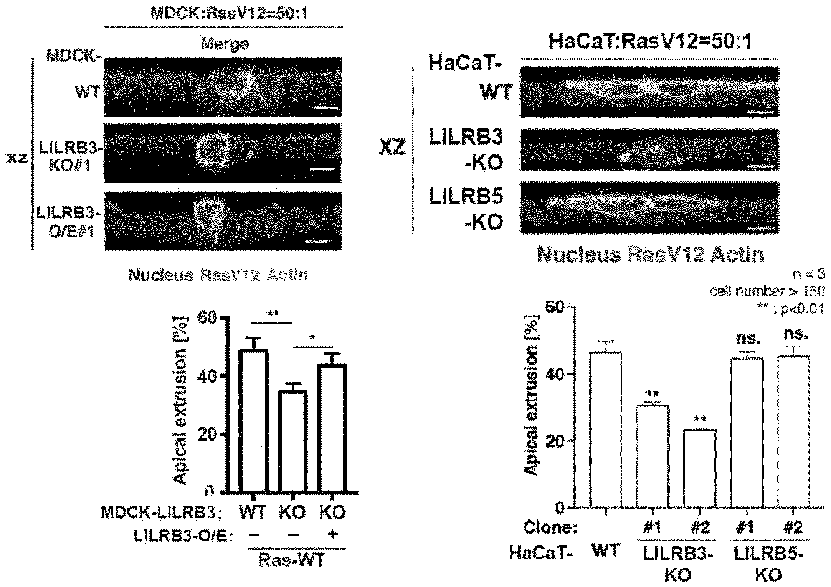

Fig. 12 Upper left figure: Deletion of normal MDCK cell-derived LILRB3 weakens apical elimination. MDCK-RasV12 cells were mixed with normal MDCK-WT or -LILRB3KO-OE on a collagen gel and, after monolayer formation, cultured with doxycycline for 24 hr. The cells were fixed and stained with phalloidin and Hoechst. Representative XZ images of normal cell and RasV12 cell. Scale bar: 10 µm. Lower left figure: quantified data of apical extrusion of RasV12. Under each experimental condition, n≥100 cells. The data shows mean±SD from 4 independent experiments. * P <0.05, ** P <0.01 (Student's t-test). Upper right figure: Knockout of LILRB3 in normal HaCaT cells suppresses apical extrusion. HaCaT-RasV12 cells were mixed with normal HaCat-WT, -LILRB3KO or -LILRB5KO cells on a collagen gel and, after monolayer formation, cultured with doxycycline for 16 hr. The cells were fixed and stained with phalloidin and Hoechst. Representative XZ images of normal cell and RasV12 cell. Scale bar: 10 µm. Lower right figure: quantified data of apical extrusion of RasV12. Under each experimental condition, n≥100 cells. The data shows mean±SD of 3 independent experiments. ** P <0.01 (Student's t-test).

### [Description of Embodiments]

### (1) regulator of the present invention

An embodiment of the present invention is a cell competition regulator, more specifically, a cell competition promoter, or a cell competition suppressor. The promoter includes a protein containing α3 domain of MHC class Ia or an agonist antibody against LILRB3, the suppressor includes a substance that inhibits binding between MHC class Ia and LILRB3, more specifically, α3 domain of MHC class Ia and D1 and D2 domains of LILRB3.

In the present specification, the "cell competition" refers to a phenomenon in which when two types of cells with different fitness (level) come close to each other in a tissue, a cell with higher fitness survives and a cell with lower fitness is eliminated. It is known that cells of the immune system such as lymphocyte and the like eliminate mutant cells. It has been elucidated that mutant cells are eliminated due to cell competition even by normal cells such as epithelial cell and the like other than immune system cells.

It has been clarified that a mechanical force via cytoskeletal molecules such as actomyosin, filamin, and vimentin functions as a driving force for cell elimination of mutant cells by cell competition. In cell competition of mutant cells by epithelial cells, these cytoskeletal factors accumulate on the normal epithelial cell side surrounding the mutant cell, whereby mutant cells are pushed out to the apical side (luminal side) of normal epithelial cells and are released. For infiltration and metastasis of mutant cells, the cells must be released to the basal side which is opposite to the apical side. Thus, the deviation of the mutant cells to the apical side is considered an antitumor phenomenon that inhibits metastasis by normal epithelial cells (Mihoko Kajita et al., pharmacological 2012; 140: 76-80). It is also known that when the cells extruded during the process of cell competition have not initiated apoptosis, programmed cell death called anoikis occurs due to the loss of attachment (VanHook M. A., Sci. Signal. 2017; Vol. 10, Issue 478, eaan5866 DOI: 10.1126/scisignal.aan5866).

In the present specification, the "eliminated cells" refers to the cells that are eliminated by losing in the cell competition of two types of cells with different fitness as the targets of the cell competition, and the "normal cell" refers to the cells that eliminate the eliminated cells by winning in the cell competition of two types of cells with different fitness as the targets of the cell competition. In the present specification, moreover, among the cells included in the eliminated cells, the cells other than the cells to be transplanted by organ transplantation, tissue transplantation, cell transplantation or the like (hereinafter to be referred to as "transplanted cells") are referred to as "abnormal cells". The abnormal cell includes mutant cells different in the fitness from normal cells due to mutations in endogenous genes or expression of foreign genes, as well as cells different in the fitness from normal cells due to epigenetic changes in gene expression. In addition, examples of the abnormal cell include, but are not limited to, cancer cells and cells with ongoing canceration (including cells in a precancerous state), cells infected with viruses and other pathogens, cells in which the pathogen infection is in progress, cells with disease-specific protein expression mutation, physically damaged cells, degenerated cells in degenerative diseases of nerve, muscle, spinal cord and other degenerative diseases, and cells with ongoing denaturation. In the present invention, a non-excluded cell is an abnormal cell when the regulation of cell competition is a stimulatory regulation. In the present invention, moreover, a non-excluded cell is a transplanted cell when the regulation of cell competition is a suppressive regulation.

In the present invention, the stimulatory regulation of cell competition is, for example, prophylaxis or treatment of cancer or virus infectious diseases, or promotion or strengthening thereof. The suppressive regulation of cell competition in the present invention includes, for example, suppression or reduction of elimination of the cells to be transplanted by organ transplantation, tissue transplantation or cell transplantation, by normal cells not belonging to an immune system.

In the present specification, cell competition is not limited to the elimination of abnormal cells and transplanted cells by an immune system cell, and may be the elimination of abnormal cells and transplanted cells by a normal cell not belonging to an immune system.

In the present specification, the "immune system cell" refers to lymphocyte such as T cell, B cell, NK (natural killer) cell, and the like, as well as cells involved in the immune function such as dendritic cell, macrophage, and the like.

In the present specification, examples of the organ to be transplanted include liver, kidney, heart, lung, pancreas, or thyroid gland, parathyroid gland, thymus, an endocrine organ including adrenal cortex or adrenal medulla, an organ containing stem cell, and the like.

In the present specification, examples of the tissue to be transplanted include skin, tissue containing stem cell, tissue containing immunocytes, and the like.

In the present specification, examples of the cell to be transplanted include bone marrow cell, non-adherent bone marrow cell, peripheral blood cell, cord blood cell, Wharton jelly-derived cell, placenta-derived cell, hair root-derived cell, adipose tissue-derived cell, lymphocyte, monocyte, macrophage and the like. Alternatively, cells selected from cells containing one or plural types of cells selected from the group consisting of bone marrow cell, non-adherent bone marrow cell, peripheral blood cell, cord blood cell, Wharton jelly-derived cell, placenta-derived cell, hair root-derived cell and adipose tissue-derived cell; and cells containing one or plural types of cells selected from the group consisting of lymphocyte, monocyte and macrophage can be mentioned.

In the present specification, examples of the neurodegenerative disease include Alzheimer's disease, mild cognitive impairment, Parkinson's disease, Huntington's disease, spinocerebellar ataxia, Creutzfeldt-Jakob disease, trauma-induced neurodegeneration, high-pressure nervous syndrome, dystonia, olivopontoserebellar atrophy, amyotrophic lateral sclerosis, multiple sclerosis, epilepsy, dementia, senile dementia, AIDS-dementia composite, AIDS-induced encephalopathy, and the like.

In the present specification, examples of the muscular degenerative disease include muscular dystrophy, distal myopathy, congenital myopathy, thyrotoxic myopathy, glycogen storage disease, mitochondrial myopathy, steroid myopathy, alcoholic myopathy, inflammatory myopathy, endocrine myopathy, lipid storage disease myopathy, myopathy associated with infections such as HIV, vitreous body myopathy, and myopathy associated with an autoimmune disease such as myasthenia gravis, and the like.

MHC is a gene family of many polymorphic proteins required for immune responses encoded by the major histocompatibility complex (MHC) region on the chromosomes of higher animals. The proteins of this MHC family play very important roles in immunity by presenting antigens and being involved in elimination of infectious pathogens such as bacteria, virus, and the like, rejection of cancer cells, rejection reaction during organ transplantation, and the like. In addition, various immune-related protein groups such as TAP (transporter associated with antigen processing) which is involved in peptide transport are also encoded in this MHC region.

MHC class I protein is present and expressed in all cells with a nucleus. The MHC class I protein binds an intracellular endogenous antigen. That is, it triggers an immune reaction by antigen presentation via MHC class I against a pathogen that proliferates in infected cells such as virus, or against a cancer antigen produced in cancer cells. MHC class I proteins are further divided into canonical class I protein (class Ia) and non-canonical class I protein (class Ib). MHC class I protein is a dimer in which a heavy chain (α chain) with a molecular weight of 45 kDa and added with a sugar chain, and a β2 microglobulin light chain with a molecular weight of 12 kDa are non-covalently bonded. A peptide antigen binds thereto and the protein is expressed on the cell surface as a trimer. The class I heavy chain consists of three extracellular domains, α1 - α3, a transmembrane domain, and an intracellular domain. A large groove-like structure exists between the α1 region and the α2 region, where the antigen is bound and presented.

Proteins in the cytoplasm, such as a pathogen that proliferates in infected cells such as virus, a protein produced in cancer cells, and the like are ubiquitinated and then degraded into peptides of about 5 - 15 amino acids by proteasome. The degraded peptide is transported into the endoplasmic reticulum (ER) by an ATP-driven transporter called TAP (transporter associated with antigen processing) on the endoplasmic reticulum (ER) membrane. The structure of TAP is of a transmembrane type and TAP is a heterodimer composed of TAP1 and TAP2. The MHC class Iα chain and β2 microglobulin are synthesized in the endoplasmic reticulum (ER), and the MHC class Iα chain, β2 microglobulin, and a peptide are bound in the endoplasmic reticulum (ER) to form an MHC-peptide complex. Thereafter, the MHC-peptide complex is placed inside a smaller endoplasmic reticulum, passes through the Golgi apparatus on its way to the cell membrane by vesicle transport, undergoes sugar chain modification, and then reaches and is expressed on the cell membrane.

In the present invention, examples of the protein containing α3 domain of MHC class Ia and involved in the regulation of cell competition include the α3 domain of MHC class I and fragments of the α3 domain of MHC class I. The fragment of the α3 domain of MHC class I may be any fragment excluding the full length of the α3 domain of MHC class I. In the present specification, the term "protein" and the term "peptide" may be used interchangeably.

Furthermore, in the present invention, examples of the aforementioned MHC class Ia include HLA-A, HLA-B, HLA-C and DLA-88, preferably HLA-B or DLA-88.

An example of the aforementioned protein containing α3 domain of MHC class Ia (α3 domain fragment protein of MHC class Ia) in the present invention is at least one protein selected from the following:
a) a protein having the amino acid sequence shown in SEQ ID NO: 1 (amino acid sequence of α3 domain of DLA88),
b) a protein having an amino acid sequence resulting from deletion, substitution, or addition of one to several amino acids in the amino acid sequence shown in SEQ ID NO: 1, and specifically binding to a protein containing D1 and D2 domains of LILRB3,
c) a protein having an amino acid sequence having at least 90% similarity to the amino acid sequence shown in SEQ ID NO: 1, and specifically binding to a protein containing D1 and D2 domains of LILRB3, and
d) a protein containing α3 domain of an orthologue of DLA-88.

In the present specification, the "similarity" means the proportion (%) of the same amino acid and similar amino acid residue to all overlapping amino acid residues in the optimal alignment where two amino acid sequences are aligned using a mathematic algorithm known in the relevant technical field (preferably, the algorithm is such that a gap can be introduced into one or both of the sequences for the optimal alignment).

The identity/similarity of the amino acid sequence in the present specification can be calculated using homology calculation algorithm NCBI BLAST (National Center for Biotechnology Information Basic Local Alignment Search Tool), and under the following conditions (expectancy =10; accept gap; matrix =BLOSUM62; filtering =OFF). Examples of other algorithm for determining the homology of amino acid sequences include the algorithm described in Karlin et al., Proc. Natl. Acad. Sci. USA, 90:5873-5877(1993) [the algorithm is incorporated in NBLAST and XBLAST program (version 2.0) (Altschul et al., Nucleic Acids Res., 25:3389-3402(1997))], the algorithm described in Needleman et al., J. Mol. Biol., 48:444-453(1970) [the algorithm is incorporated in GAP program in the GCG software package], the algorithm described in Myers and Miller, CABIOS, 4:11-17(1988) [the algorithm is incorporated in ALIGN program (version 2.0) which is a part of the CGC sequence alignment software package], the algorithm described in Pearson et al., Proc. Natl. Acad. Sci. USA, 85:2444-2448(1988) [the algorithm is incorporated in FASTA program in the GCG software package] and the like, and these can similarly be used preferably.

The protein containing α3 domain of class Ia of the present invention may be, as long as it specifically binds to a protein containing D1 and D2 domains of LILRB3, a protein containing or consisting of an amino acid sequence resulting from the deletion of 1 - 20, preferably 1 - 10, more preferably 1 - several (5, 4, 3 or 2) amino acids from the amino acid sequence of the protein containing the α3 domain (e.g., amino acid sequence of α3 domain of DLA88 shown in SEQ ID NO: 1), an amino acid sequence resulting from the addition of 1 - 20, preferably 1 - 10, more preferably 1 - several (5, 4, 3 or 2) amino acids to the amino acid sequence of the protein containing the α3 domain (e.g., amino acid sequence shown in SEQ ID NO: 1), an amino acid sequence resulting from the substitution of 1 - 20, preferably 1 - 10, more preferably 1 - several (5, 4, 3 or 2) amino acids in the amino acid sequence of the protein containing the α3 domain (e.g., amino acid sequence shown in SEQ ID NO: 1) with other amino acids, or a combination of such amino acid sequences. Substitution of amino acids with similar properties (e.g., glycine and alanine, valine and leucine and isoleucine, serine and threonine, aspartic acid and glutamic acid, asparagine and glutamine, lysine and arginine, cysteine and methionine, phenylalanine and tyrosine, etc.) can afford a larger number of substitutions, and the like.

The protein containing α3 domain of class Ia of the present invention may be, as long as it specifically binds to a protein containing D1 and D2 domains of LILRB3, a protein containing or consisting of an amino acid sequence having a similarity of not less than 80%, preferably not less than 85% (not less than 86%, 87%, 88%, or 89%), more preferably not less than 90% (not less than 91%, 92%, 93%, or 94%), further preferably not less than 95% (not less than 96%, 97%, or 98%), further more preferably not less than 99%, with the amino acid sequence (e.g., the amino acid sequence shown in SEQ ID NO: 1) of the protein containing α3 domain of the class Ia.

In the present invention, the agonist antibody against LILRB3 is not particularly limited as long as it is an antibody capable of binding to LILRB3 (more specifically, D1 and D2 domains of LILRB3) and regulating cell competition (more specifically, promoting cell competition). The antibody can be appropriately screened for and obtained by those of ordinary skill in the art based on the methods described in, for example, WO 2003/091424, WO 2005/056602, and the like.

LILRB3 which functions as a receptor for MHC class Ia is also called ENSCAFG00000028453, CD85a, ILT5, or LIR3. LILRB3 is a type of immunoglobulin-like receptors, has extracellular immunoglobulin domains of D1-D4, transmembrane domains, and intracellular domains, and is well known for expression in professional immune cells. It is also expressed in epithelial cells. However, the function thereof has not been sufficiently elucidated.

In the present specification, examples of the protein containing D1 and D2 domains of LILRB3 include the D1 and D2 domains of LILRB3 and fragments of the D1 and D2 domains of LILRB3. The fragment of the D1 and D2 domains of LILRB3 may be any fragment excluding the full length of the D1 and D2 domains of LILRB3.

In the present invention, the orthologue of DLA-88 (protein consisting of the full-length amino acid sequence of DLA-88 shown in SEQ ID NO: 13), and the orthologue of dog LILRB3 (ENSCAFG00000028453) (protein consisting of the full-length amino acid sequence of ENSCAFG00000028453 shown in SEQ ID NO: 14) may be, for example, those in mammals other than dog, preferably primates (e.g., ape, human, etc.), more preferably human (e.g., SEQ ID NO: 11: amino acid sequence of α3 domain of human MHC Ib, SEQ ID NO: 15: full-length amino acid sequence of human MHC B, SEQ ID NO: 12: amino acid sequence of D1 and D2 domains of human LILRB3, SEQ ID NO: 16: full-length amino acid sequence of human LILRB3).

An example of the aforementioned protein containing D1 and D2 domains of LILRB3 in the present invention is at least one protein selected from the following:
e) a protein having the amino acid sequence shown in SEQ ID NO: 8 (amino acid sequence of D1 and D2 domains of ENSCAFG00000028453),
f) a protein having an amino acid sequence resulting from deletion, substitution, or addition of one to several amino acids in the amino acid sequence shown in SEQ ID NO: 8, and specifically binding to a protein containing α3 domain of MHC class Ia,
g) a protein having an amino acid sequence having at least 80% similarity to the amino acid sequence shown in SEQ ID NO: 8, and specifically binding to a protein containing α3 domain of MHC class Ia, and
h) a protein containing D1 and D2 domains of an orthologue of ENSCAFG00000028453.

The protein containing D1 and D2 domains of LILRB3 of the present invention may be, as long as it specifically binds to a protein containing α3 domain of MHC class Ia, a protein containing or consisting of an amino acid sequence resulting from the deletion of 1 - 20, preferably 1 - 10, more preferably 1 - several (5, 4, 3 or 2), amino acids from the amino acid sequence of the protein containing the D1 and D2 domains (e.g., amino acid sequence of D1 and D2 domains of ENSCAFG00000028453 shown in SEQ ID NO: 8), an amino acid sequence resulting from the addition of 1 - 20, preferably 1 - 10, more preferably 1 - several (5, 4, 3 or 2), amino acids to the amino acid sequence of the protein containing the D1 and D2 domains (e.g., amino acid sequence shown in SEQ ID NO: 8), an amino acid sequence resulting from the substitution of 1 - 20, preferably 1 - 10, more preferably 1 - several (5, 4, 3 or 2), amino acids in the amino acid sequence of the protein containing the D1 and D2 domains (e.g., amino acid sequence shown in SEQ ID NO: 8) with other amino acids, or a combination of such amino acid sequences. Substitution of amino acids with similar properties (e.g., glycine and alanine, valine and leucine and isoleucine, serine and threonine, aspartic acid and glutamic acid, asparagine and glutamine, lysine and arginine, cysteine and methionine, phenylalanine and tyrosine, etc.) can afford a larger number of substitutions, and the like.

The protein containing D1 and D2 domains of LILRB3 of the present invention may be, as long as it specifically binds to a protein containing α3 domain of MHC class Ia, a protein containing or consisting of an amino acid sequence having a similarity of not less than 80%, preferably not less than 85% (not less than 86%, 87%, 88%, or 89%), more preferably not less than 90% (not less than 91%, 92%, 93%, or 94%), further preferably not less than 95% (not less than 96%, 97%, or 98%), further more preferably not less than 99%, with the amino acid sequence (e.g., the amino acid sequence shown in SEQ ID NO: 8) of the protein containing D1 and D2 domains of LILRB3.

The protein containing α3 domain of MHC class Ia and the protein containing D1 and D2 domains of LILRB3 to be used in the present invention may also be a salt, a hydrate, a solvate thereof, or the like.

The "substance that inhibits binding between MHC class Ia and LILRB3" to be used in the present invention (hereinafter sometimes to be abbreviated as "the substance of the present invention") is not particularly limited as long as the binding between MHC class Ia and LILRB3, more specifically, the binding between a protein containing α3 domain of MHC class Ia, and D1 and D2 domains of LILRB3 (expressed in a cell), is inhibited. Specifically, for example, a protein containing D1 and D2 domains of LILRB3 (including dominant negative variant, and the like), an antibody against D1 and D2 domains of LILRB3 (including neutralizing antibody, and the like), aptamer, an inhibitor of LILRB3 expression and the like can be mentioned.

Antibodies to the D1 and D2 domains of LILRB3 to be used in the present invention can be obtained as polyclonal or monoclonal antibodies by means known per se. Alternatively, commercially available products may also be used. The origin of the antibody to be used in the present invention is not particularly limited, and a mammal-derived antibody is preferred, and a human-derived antibody is more preferred. A mammal-derived monoclonal antibody may be either one produced in a hybridoma or one produced in a host transformed with an expression vector containing an antibody gene by a genetic engineering technique. Antibody-producing hybridoma can be produced by a method known per se. For example, using the D1 and D2 domains of LILRB3 or a part thereof as an antigen, the antigen is used for immunization according to a conventional immunization method, and the obtained immunocytes are fused with a known parent cell by a conventional cell fusion method. Then, the hybridoma can be produced by screening monoclonal antibody-producing cells by a conventional screening method.

The aptamer to be used in the present invention may be a nucleic acid aptamer or a peptide aptamer. In the case of a nucleic acid aptamer, the nucleic acid may be DNA, RNA, or a DNA/RNA chimera. In addition, it may be a nucleic acid or peptide in which ribose, phosphate skeleton, nucleic acid base, amino acid residue, and both terminal portions are modified. The nucleic acid aptamer may be double-stranded or single-stranded, preferably single-stranded. The aptamer in the present invention can be selected using a method well known to those of ordinary skill in the art. Unlimitatively, for example, it can be selected by the SELEX method (Systematic Evolution of Ligands by Exponential Enrichment) (Tuerk, C. and Gold, L., 1990, Science, 249: 505-510) or the Two-hybrid method for yeast.

An inhibitor of LILRB3 expression to be used in the present invention may act at any stage of transcription level, post-transcriptional regulation level, protein translation level, post-translational modification level, and the like of the gene encoding LILRB3. Therefore, examples of the inhibitor of LILRB3 expression include a nucleic acid that inhibits transcription of the gene encoding LILRB3 (e.g., antigene), a nucleic acid that inhibits processing of initial transcription product into mRNA, a nucleic acid that inhibits translation of mRNA into protein (e.g., antisense nucleic acid, miRNA) or degrades mRNA (e.g., siRNA, ribozyme, micro RNA (miRNA)), and the like.

SiRNA can be designed based on the cDNA sequence information of the LILRB3 gene, for example, according to the rules proposed by Elbashir et al. (Genes Dev., 15, 188-200 (2001)). A short hairpin RNA (shRNA), which is a precursor of siRNA, can be designed by appropriately selecting any linker sequence (for example, about 5 - 25 bases) capable of forming a loop structure and linking the sense strand and antisense strand of siRNA via the linker sequence.

The siRNA and/or shRNA sequences can be searched for using search software provided free of charge on various websites. Examples of such site include, but are not limited to, siDESIGN Center (http://dharmacon.horizondiscovery.com/jp/design-center/?rdr=true&LangType=1041&pageid=17179928204) provided by Dharmacon, siRNA Target Finder(https://www.genscript.com/tools/sirna-target-finder) provided by GenScript, and the like.

The miRNA can be searched for using target prediction software provided free of charge on various websites. Examples of such site include, but are not limited to, TargetScan (http://www.targetscan.org/vert_72/) published by the Whitehead Institute in the United States, DIANA-micro-T-CDS (http://diana.imis.athena-innovation.gr/DianaTools/index.php?r=microT_CDS/index) published by the Alexander Fleming Center for Biomedical Sciences, Greece, and the like. Alternatively, miRNA that targets mRNA encoding LILRB3 can also be searched for using TarBase (http://carolina.imis.athena-innovation.gr/diana_tools/web/index.php?r=tarbasev8/index), which is a database relating to miRNA that has been experimentally proven to act on target mRNA, published by the Pasteur Institute of the University of Thessaly, and the like.

The siRNA can be prepared by synthesizing the sense strand and antisense strand of the target sequence on mRNA by a DNA/RNA automatic synthesizer, and denaturing them in an appropriate annealing buffer at about 90 - about 95°C for about 1 min, and annealing them at about 30 - about 70°C for about 1 - about 8 hr. It can also be prepared by synthesizing shRNA to be a precursor of siRNA, and cleaving same with a dicer. miRNA and pre-miRNA can be synthesized by a DNA/RNA automatic synthesizer based on the sequence information thereof.

The antisense nucleic acid may be DNA, RNA, or a DNA/RNA chimera. When the antisense nucleic acid is DNA, the RNA:DNA hybrid formed by the target RNA and the antisense DNA can be recognized by endogenous RNase H and cause selective degradation of the target RNA. The length of the target region of the antisense nucleic acid is not particularly limited as long as the hybridization of the antisense nucleic acid results in the inhibition of translation into a protein. It may be a full sequence or a partial sequence of the mRNA encoding the protein, where a short one may be about 10 bases, and a long one may be the full sequence of mRNA or the initial transcription product. In addition, the antisense nucleic acid may be one that can not only hybridize with target the mRNA and initial transcription product to inhibit translation into a protein, but can also bind to these genes, which are double-stranded DNAs, to form a triplex and inhibit transcription into RNA (antigene).

The antisense nucleic acid can be prepared by determining the target sequence of mRNA or initial transcription product based on the cDNA sequence or genomic DNA sequence of the target gene, and synthesizing a sequence complementary to the sequence by using a commercially available DNA/RNA automatic synthesizer.

The regulator of the present invention is prepared and used as a parenteral (e.g., intravenous, intraarterial, intramuscular, subcutaneous, intradermal, intraperitoneal, vaginal, intramuscular, transnasal, intrarectal, intraoral, intraocular, intra-aural, sublingual, etc.) preparation, or oral preparation. When administered orally, it may be administered before, after, or between meals. The regulator of the present invention is prepared and used as, for example, a preparation prepared by diluting with a solvent such as physiological saline and the like to a predetermined concentration and dose and adding a pharmaceutically acceptable additive. The regulator of the present invention may also be prepared as a pharmaceutical composition.

As the dosage form of the regulator of the present invention, liquids such as injection, cream, ointment, drink, aerosol, skin gel, eye drop, nasal drop and the like; solid agents such as tablet, capsule, powder, powder preparation, granule, tablet, sustained-release preparation, suppository and the like; and the like in which an effective amount of a protein containing α3 domain of MHC class Ia, or a substance that inhibits binding between MHC class Ia and LILRB3is dissolved, dispersed or emulsified in a dilution solution or dispersion medium such as water, saline and the like can be used. These may be in the form of an inclusion in which the active ingredient is encapsulated in a liposome, a sustained-release material, or the like, a support form in which the active ingredient is supported on a carrier, or the like. The dosage form of the regulator of the present invention may be a preparation in a unit-dose form or a multiple-dose form.

Examples of the oral preparation include tablet, powder, powder preparation, granule, fine granule, pill, capsule, troche, chewable agent, liquid, emulsion, microcapsule, suspension, elixir, syrup, sustained-release preparation and the like. These may be in the form of an inclusion in which the active ingredient is encapsulated in a liposome, a sustained-release material, or the like, a support form in which the active ingredient is supported on a carrier, or the like.

As the parenteral agent, liquids such as injection, transfusion, cream, ointment, aerosol, skin gel, eye drop, nasal drop and the like in which an effective amount of adrenomedulin or a modified product thereof, or a derivative thereof, or a salt thereof is dissolved, dispersed or emulsified in a dilution solution or dispersion medium such as water, saline and the like can be mentioned. Alternatively, it may be in the form of powder preparation, transdermal patch, lotion, ointment, cataplasm or suppository.

The aforementioned pharmaceutically acceptable pharmaceutical product additive can be prepared by adding additives known to those of ordinary skill in the art, such as stabilizer, antioxidant, pH adjuster, buffering agent, suspending agent, emulsifier, surfactant and the like. The kind, usage, and dosage of these pharmaceutical additives are described in the Pharmaceutical Additives Dictionary 2007 (edited by the Japan Pharmaceutical Additives Association, Yakuji Nippo Co., Ltd., July 2007), and the like, and the additives can be prepared and used in accordance with these descriptions.

Specifically, for example, organic acids such as tartaric acid, citric acid, succinic acid, fumaric acid and the like can be used as a stabilizer, ascorbic acid, dibutyl hydroxytoluene, propyl gallate and the like can be used as an antioxidant, dilute hydrochloric acid or sodium hydroxide aqueous solution and the like can be used as a pH adjuster, citric acid, succinic acid, fumaric acid, tartaric acid or ascorbic acid or a salt thereof, glutamic acid, glutamine, glycine, aspartic acid, alanine or arginine or a salt thereof, magnesium oxide, zinc oxide, magnesium hydroxide, phosphoric acid or boric acid or a salt thereof can be used as a buffering agent, lecithin, sucrose fatty acid ester, polyglycerol fatty acid ester, polyoxyethylene hydrogenated castor oil, polysorbate or polyoxyethylene. polyoxypropylene copolymer substance and the like can be used as a suspending agent or emulsifier, polysorbate 80, sodium lauryl sulfate, polyoxyethylene hydrogenated castor oil and the like can be used as a surfactant, though the examples are not limited thereto.

The subject of administration of the regulator of the present invention includes human, mammals other than human (e.g., mouse, rat, hamster, rabbit, cat, dog, bovine, sheep, monkey, etc.), and the like. When applied to mammals other than human, the dose of the regulator of the present invention may be appropriately adjusted according to the body weight and size of the animal.

Regarding the regulator of the present invention, the dose per day for adults of a protein containing the α3 domain of MHC class Ia, an agonist antibody against LILRB3, or a substance that inhibits the binding between MHC class Ia and LILRB3, each of which is an active ingredient, can be appropriately adjusted according to gender, age, body weight, symptoms thereof, administration route, dosage form and the like. For example, when the active ingredient is a protein, the dose can be generally selected within the range of about 0.0001 mg/kg - about 1,000 mg/kg. Alternatively, the dose can be generally selected within the range of, for example, about 0.001 mg/body - about 1,00000 mg/body, per subject (patient). However, the regulator of the present invention is not limited by the above-mentioned doses.

The above-mentioned daily dose of the regulator of the present invention may be administered at once or in multiple divided doses. The timing of administration may be before, after, or between meals. While the administration interval is not particularly limited, it may be administered daily, every other day, or intermittently. While the administration period is not particularly limited, long-term administration is possible.

When the cell competition regulator of the present invention promotes cell competition, the cells eliminated by the cell competition (e.g., mutant cells, etc.) are caspase-positive cells, as shown in Examples described later, and cell death (e.g., apoptosis) can be induced. Therefore, the regulator used alone is suitable for the prophylaxis or treatment of cancer (including precancer state). In addition, cells involved in cancer metastasis or recurrence can be eliminated by the regulator even after cancer treatment (e.g., surgical treatment). The eliminated cells are caspase-positive cells as described above, and cell death (e.g., apoptosis) can be induced. Therefore, the regulator used alone is also suitable for the prophylaxis or treatment of cancer metastasis or recurrence.

Furthermore, as described above, it is highly possible that cell death (e.g., apoptosis) has been induced in the cells eliminated by the cell competition regulator of the present invention. For example, even in cancers that are resistant to immune checkpoint inhibitors (e.g., anti-PD-1 antibody, etc.), a more superior prophylactic or therapeutic effect on cancer can be expected by using the cell competition regulator of the present invention in combination with the below-mentioned medicaments.

When the cell competition regulator of the present invention suppresses cell competition, it is suitable for, for example, maintaining the transplanted cells in a desired place, as will be understood from the Examples described later. Furthermore, for example, to further increase the efficiency of cell transplantation, it may be used in combination with the below-mentioned medicaments.

As described above, the regulator of the present invention may be used concurrently with other medicaments and/or other treatment methods (concomitant drug), that is, may be used in combination. When used in combination, the regulator of the present invention and other medicament may be provided in the form of a single drug, or provided in the form of a pharmaceutical combination or kit containing a plurality of preparations formulated separately. The administration period of the drug used in combination with the regulator of the present invention is not limited. The drug used in combination with the regulator of the present invention may be simultaneously or separately (e.g., continuously, at intervals, etc.) administered to the administration subject. The dose of the medicament to be used in combination may be determined according to the dose clinically used, and can be appropriately selected depending on the subject of administration, administration route, disease, combination and the like.

When the regulator of the present invention is a cell competition promoter, the aforementioned other medicament may be, for example, an anticancer agent or an antiviral agent. When the regulator of the present invention is a cell competition suppressor, the other medicament may be an immunosuppressant, a rejection suppressant for transplanted organ, and/or a post-transplantation engraftment promoter.

In the cell competition promoter of the present invention, examples of the anticancer agent as the aforementioned other medicament include, but are not limited to, alkylating agent, cytotoxic antibiotic, platinum preparation, antimetabolite, kinase inhibitor, angiogenesis inhibitor, hormonal agent, DNA modifying enzyme inhibitor, proteosome inhibitor, alkaloid agent, type I and type II topoisomerase inhibitor, histone deacetylase inhibitor, cytokine preparation, hormonal agent, immune checkpoint inhibitor, natural killer cell activator, indoleamine 2,3-dioxygenase (IDO) inhibitor, monoclonal antibody, and other molecular-targeted therapeutic agents.

More specific examples of the aforementioned anticancer agent include, but are not limited to, doxorubicin, daunorubicin, cisplatin, oxaliplatin, carboplatin, paclitaxel, irinotecan, SN-38, actinomycin D, vincristine, vinblastine, methotrexate, azathioprine, fluorouracil, mitomycin C, docetaxel, cyclophosphamide, capecitabine, epirubicin, gemcitabine, mitoxantrone, leucovorin, vinorelbine, trastuzumab, etoposide, estramustine, prednisone, interferon α, interleukin-2, bleomycin, ifosfamide, mesna, altretamine, topotecan, cytarabine, methylprednisolone, dexamethasone, mercaptopurine, thioguanine, fludarabine, gemtuzumab, idarubicin, mitoxantrone, tretinoin, alemtuzumab, chlorambucil, cladribine, imatinib, epirubicin, dacarbazine, procarbazine, mechlorethamine, rituximab, denileukin diftitox, trimethoprim/sulfamethoxazole, allopurinol, carmustine, tamoxifen, filgrastim, temozolomide, melphalan, vinorelbine, azacytidine, thalidomide, mitomycin, legolafenib, cetuximab, panitumumab, ramsilmab, gefitinib, erlotinib, afacinib, crizotinib, alectinib, ceritinib, lenvatinib, lapatinib, pertuzumab, sunitinib, sorafenib, axitinib, pazopanib, nivolumab, pembrolimazab, ipilimumab, vemurafenib, everolimus, temsirolimus, bevacizumab, geldanamycin and the like.

The cell competition promoter of the present invention, and the antiviral agent as the aforementioned other medicament include interferon, nucleoside and nucleotide reverse transcriptase inhibitor, non-nucleoside reverse transcriptase inhibitor, protease inhibitor, integrase inhibitor, fusion inhibitor, mature inhibitor, guanosine analog, pridin analog, pyrimidine analog, and other "unclassified" antiviral agent recognized in the art and not in any of the above-mentioned classes (e.g., foscarnet and miltefosine); however, examples are not limited to these.

More specific examples of the aforementioned antiviral agent include, but are not limited to, abacavir, acyclovir, adefovir, amantadine, amdoxovir, amprenavir, aplaviroc, apricitabine, arbidol, atazanavir, bevirimat, BMS-488043, boceprevir, brivudine, cidofovir, DCM205, docosanol, delavirdine, didanosine, darunavir, efavirenz, elvitegravir, elvucitabine, emtricitabine, enfuvirtide, epigallocatechin gallate, etravirine, famciclovir, fosamprenavir, gancyclovir, globoidnan A, griffithsin, ibalizumab, idoxuridine, indinavir, lamivudine, lopinavir, loviride, maraviroc, nelfinavir, nevirapine, oseltamivir, pegylated interferon α-2a, pegylated interferon α-2b, penciclovir, peramivir, plerixafor, pro 140, racivir, raltegravir, ritonavir, ribavirin, rimantadine, rilpivirine, saquinavir, stampidine, stavudine, tenofovir, tipranavir, tnx-355, trifluridine, tromantadine, valaciclovir, valganciclovir, vicriviroc, vidarabine, viramidine, vivecon, zalcitabine, zanamivir, zidovudine and the like.

In the cell competition regulator of the present invention, the immunosuppressant as the aforementioned other medicament includes, but are not limited to, steroid, cyclosporine, cyclosporine analogue, cyclophosphamide, methylprednisone, prednisone, azathioprine, tacrolimus hydrate, 15-deoxyspergualin, natalizumab, rapamycin, or etanercept.

In the present invention, the dose of the above-mentioned concomitant drug can be set to any amount within the range where side effects do not pose a problem. The daily dose of the medicament to be used in combination varies depending on the degree of symptoms, age, gender, body weight, sensitivity difference of the subject of administration, timing and interval of administration, nature of pharmaceutical preparation, pharmacy, kind, the kind of active ingredient, and the like, and is not particularly limited.

### (2) regulation method and the like of the present invention

The other embodiment of the present invention is a method for regulating cell competition, comprising administering a therapeutically effective amount of a protein containing α3 domain of MHC class Ia or an agonist antibody against LILRB3, or a substance that inhibits binding between MHC class Ia and LILRB3 (more specifically, binding between α3 domain of MHC class Ia and D1 and D2 domains of LILRB3) to a subject in need thereof.

In the method for regulating cell competition, when a protein containing α3 domain of MHC class Ia or an agonist antibody against LILRB3 is administered, the regulation method may afford prophylaxis or treatment of cancer or virus infectious diseases.

Furthermore, in the method for regulating cell competition, when a substance that inhibits binding between MHC class Ia and LILRB3 is administered, the regulation method may improve an engraftment failure in organ transplantation, tissue transplantation, or cell transplantation, or suppress progression of symptoms of a neurodegenerative disease, a muscular degenerative disease, or other various denaturation diseases.

A further embodiment of the present invention is use of a protein containing α3 domain of MHC class Ia or an agonist antibody against LILRB3, or a substance that inhibits binding between MHC class Ia and LILRB3 in the production of a medicament for regulating cell competition.

The contents for the regulator of the present invention described in the above-mentioned (1) can be referred to for the above-mentioned regulation method and the like of the present invention.

All references referred to in the present specification are incorporated herein by reference in their entirety. The detailed description and Examples in the present specification illustrate embodiments of the present invention and should not be construed as limiting the scope of rights of the present invention.

### [Example 1]

Cloning, genome editing, cell culture, microscopic observation, protein expression, induction, purification, and the like in the Examples of the present specification were performed according to the manufacturer's instructions and using commercially available vectors, reagents, kits, and the like.

### <Suppression of the ability of MDCK normal cell to eliminate abnormal cell by suppression of antigen presentation in RasV12-expressing cell>

### 1. Experiment method

### Establishment of RasV12-expressing cell

PTRE3G-GFP-RasV12 and Hyper PiggyBac transposase expression vector were introduced into MDCK cells of a cell line derived from canine renal tubular epithelial cell by Nucleofection. The MDCK cells with gene transfer were selected by Blasticidin and the surviving cell colonies were isolated to obtain a monoclonal pTRE3G-GFP-RasV12-expressing MDCK stable cell line. At 24 hr after the addition of additional doxycycline (200 ng/mL), and using the presence or absence of fluorescence of GFP under a fluorescence microscope as an index, RasV12-expressing MDCK cells in which GFP-RasV12 was expressed more uniformly in all cells were finally obtained as a usable stable cell line. In the following Examples, the aforementioned RasV12-expressing MDCK cells were used as abnormal cells, and cell competition with normal MDCK cells was examined.

### Establishment of TAP1 gene-deficient RasV12-expressing cell

A pCDH-QC-sgRNA (Maruyama et al., Nat. Biotechnol.2015) in which the guide sequence (target sequence (gRNA)) shown in SEQ ID NO: 3 was inserted was introduced into a tetracycline-inducible Cas9 stable MDCK cell line (pCW-Cas9-MDCK).

After gene transfer, Cas9 was induced with doxicycline (200 ng/mL). Furthermore, only the cells having pCDH-QC-sgRNA were selected by culturing in the presence of hygromycin (400 ug/mL) for 7 days. The selected cells were subjected to limiting dilution to achieve a concentration of 0.5 cell per well to give a single clone. Thereafter, by sequence analysis, a clone with gene deficiency was used as a KO cell of the TAP1 gene. To eliminate the clonal effect, cells of two clones (TAP-KO#1 and TAP-KO#2) were selected, and TAP1 gene-deficient RasV12-expressing MDCK cells were established using PB-pTRE3G-RasV12 vector by an operation similar to the above-mentioned RasV12-expressing cell establishment.

### Evaluation of cell non-autonomous effect between normal cell and abnormal cell

Type I-A Collagen, 5X DMEM solution, and pH adjustment buffer solution were mixed on ice at a ratio of 7:2:1 to prepare a collagen gel solution. A 15 mm coverslip was placed in a 12-well plate, 500 µL of collagen gel solution was placed therein, and the mixture was allowed to stand at 37°C for 30 min to harden the gel solution.

MDCK normal cells and RasV12-expressing MDCK cells (TAPl gene wild-type cells (TAP-WT-RasV12) or TAP1 gene-deficient cells (TAP-KO#1-RasV12 or TAP-KO#2-RasV12)) were mixed at a ratio of 50:1 by mixing 1×10⁶ cells and 2×10⁴ cells thereof, respectively, seeded on a collagen gel, cultured at 37°C for 8-12 hr until a monolayer was formed, and then a doxycycline treatment (200 ng/mL) was performed for 24 hr.

After 24 hrs of doxycycline treatment, 4% Paraformaldehyde/PBS was added, and the cells were incubated for 15 min at room temperature in shading to fix the cells. After fixation, 0.1% Triton X-100/PBS was added, and the mixture was incubated at room temperature for 15 min in shading, and subjected to a membrane permeation treatment. Blocking was performed by allowing the mixture to stand at room temperature for 1 hr in shading with 1% BSA/PBS. Successively, the mixture was reacted with Alexa-Fluor-568-conjugated phalloidin diluted 200-fold with 1% BSA/PBS at room temperature for 1 hr. After further washing with PBS for 5 min×3 times, the mixture was reacted with Hoechst 33342 diluted 4,000-fold with PBS at room temperature for 10 min. Finally, the mixture was sealed on a slide glass using Mowiol.

Filamin accumulation was statistically processed using an open source ImageJ (Fig. 1B). As the elimination efficiency of RasV12-expressing cells (TAP-WT-RasV12, TAP-KO#1-RasV12 and TAP-KO#2-RasV12) resulting from the accumulation of Filamin, colonies of GFP fluorescence-positive cells that consisted of 2 to 8 cells were found under a confocal microscope, the number of cells that escaped to the apical side from among them was counted, and finally, the ratio of the number of cells that escaped to the total number of RasV12-expressing cells was shown.

To examine whether antigen presentation of RasV12-expressing MDCK cells promotes the elimination ability of normal MDCK cells, the elimination efficiency of TAP1 gene wild-type RasV12-expressing cells (TAP-WT-RasV12) by MDCK normal cells was compared with the elimination efficiency of TAP1 gene-deficient RasV12-expressing cells (TAP-KO#1-RasV12 and TAP-KO#2-RasV12) by normal MDCK cells.

### 2. Experiment results

When RasV12-expressing cells (TAP-WT-RasV12, TAP-KO#1 or TAP-KO#2) and MDCK normal cells were co-cultured, the accumulation of Filamin on the MDCK normal cell side was confirmed by the cell non-autonomous effect between normal cells and abnormal cells (Fig. 1A). On the other hand, the accumulation of Filamin in MDCK normal cells surrounding TAP1 gene-deficient RasV12-expressing cells (TAP-KO#1 and TAP-KO#2) was statistically more significantly suppressed than the Filamin accumulation in MDCK normal cells surrounding TAP1 gene wild-type RasV12-expressing cells (TAP-WT-RasV12) (Fig. 1B). The elimination efficiency of RasV12-expressing cells resulting from the accumulation of Filamin was also examined. As a result, it was confirmed that the elimination efficiency in TAP1 gene-deficient RasV12-expressing cells (TAP-KO#1-RasV12 and TAP-KO#2-RasV12) was suppressed as compared with the elimination efficiency in RasV12-expressing cells (TAP-WT-RasV12) (Fig. 1C, 1D).

### [Example 2]

### <Positive regulation of promotion of elimination of abnormal cell by DLA88 of DLA family>

### 1. Experiment method

### Establishment of DLA88-KO cell, DLA79-KO cell, DLA64-KO cell and DLA12-KO cell

DLA12, DLA64, DLA79 and DLA88 of the DLA family, which are orthologs of MHC class I protein in dogs, were focused as proteins involved in antigen presentation. A pCDH-QC-sgRNA (Maruyama et al., Nat. Biotechnol.2015) in which the guide sequences (target sequence (gRNA)) shown in SEQ ID NO: 4 - 7 (DLA88, DLA79, DLA64, and DLA 12, respectively) were inserted was introduced into a tetracycline-inducible Cas9 stable MDCK cell line (pCW-Cas9-MDCK).

After gene transfer, Cas9 was induced with doxicycline (200 ng/mL). Furthermore, only the cells having pCDH-QC-sgRNA were selected by culturing in the presence of hygromycin (400 µg/mL) for 7 days. The selected cells were subjected to limiting dilution to achieve a concentration of 0.5 cell per well to give a single clone. Thereafter, by sequence analysis, a clone with gene deficiency was used as a KO cell of each gene. In the following, DLA88 gene-deficient RasV12-expressing cell, DLA79 gene-deficient RasV12-expressing cell, DLA64 gene-deficient RasV12-expressing cell, and DLA12 gene-deficient RasV12-expressing cell are referred to as DLA88-KO-RasV12, DLA79-KO-RasV12, DLA64-KO-RasV12, and DLA12-KO-RasV12, respectively.

To eliminate the clonal effect, cells of two clones for each gene-deficient RasV12-expressing cell were selected, and gene-deficient RasV12-expressing cells were established using PB-pTRE3G-RasV12 vector by an operation similar to the above-mentioned RasV12-expressing cell establishment.

In the same manner as in Example 1, the accumulation of Filamin was measured, and the elimination efficiency of abnormal cells (DLA88-KO-RasV12, DLA79-KO-RasV12, DLA64-KO-RasV12 and DLA12-KO-RasV12) resulting from the accumulation of Filamin was calculated. In the same manner as in Example 1, the cells were fixed and observed with a fluorescence microscope.

### 2. Experiment results

As a result of gene deletion of DLA12, DLA64, DLA79 and DLA88 by the CRISPR/Cas9 system, the elimination efficiency by MDCK normal cells was suppressed only in DLA88 gene-deficient RasV12-expressing cells (DLA88-KO-RasV12) (Fig. 2A). This indicates that DLA88 positively regulates the elimination of RasV12-expressing cells. Furthermore, the behavior of abnormal cells (DLA88-KO-RasV12, DLA79-KO-RasV12, DLA64-KO-RasV12 and DLA12-KO-RasV12) was analyzed with a confocal microscope. As a result, the DLA88 gene-deficient RasV12-expressing cell (DLA88-KO-RasV12) showed basal extrusion that penetrates into the basal side rather than the apical extrusion seen in RasV12-expressing cells (DLA-WT-RasV12, DLA79-KO-RasV12, DLA64-KO-RasV12 and DLA12-KO-RasV12) free of the lack of DLA88 gene (Fig. 2B).

### [Example 3]

<Promotion of elimination of abnormal cell by extracellular domain protein of DLA88 >

### 1. Experiment method

### Production of recombinant protein

### DLA88 α(alpha)3

The extracellular domain of DLA88 is composed of three α domains (Fig. 3A). Therefore, a recombinant protein of α1 and α2 domains (DLA88-alpha1/2) and a recombinant protein of α3 domain (DLA88-alpha3) were produced. To produce the recombinant protein of DLA88-alpha3 (SEQ ID NO: 1) and the recombinant protein of DLA88-alpha1/2, the expression plasmids pGEX-6p-1-DLA88-alpha3 and pGEX-6p-1-DLA88-alpha1/2 were prepared by inserting the DNA fragment encoding DLA88-alpha3 (SEQ ID NO: 2) and the DNA fragment encoding DLA88-alpha1/2 into the EcoRI/XhoI restriction enzyme site of pGEX-6p-1.

IPTG (final concentration 0.1 mM) was added to the culture medium of BL21 transformed with pGEX-6p-1-DLA88-alpha3 or DLA88-alpha1/2, and the expression of the recombinant protein was induced at 25°C for 2 hr. The harvested *Escherichia coli* was solubilized, and a recombinant protein of DLA88-alpha3 (SEQ ID NO: 1) and DLA88-alpha1/2 was purified from the extract by using Gluthatione Sepharose 4B.

### Evaluation of cell non-autonomous effect between normal cell and abnormal cell

In the same manner as in Example 1 except that a recombinant protein treatment (30 nM or 300 nM) was performed in addition to the doxycycline treatment, a cell non-autonomous effect was evaluated.

### 2. Experiment results

The extracellular domain of DLA88 is composed of three α domains. Therefore, a recombinant protein of the α3 domain was produced. In addition, after mixing at a cell ratio of normal cells:mutant cells=50:1 to form a single cell layer, the GFP-RasV12 protein was expressed with doxycycline. Treatment with the recombinant protein DLA88-α3 simultaneously with doxycycline promoted the elimination of mutant cells in a concentration-dependent manner (Fig. 3A and Fig. 3B). In addition, even under the condition of normal cells:DLA88-deficient mutant cells=50:1, elimination of abnormal cells was promoted in the presence of the recombinant protein. From this, DLA88 promotes elimination of abnormal cells, and the recombinant protein in the extracellular domain promotes elimination of abnormal cells (Fig. 3C, Fig. 3D).

### [Example 4]

### Knockout of LILRB3

### 1. Experiment method

To knock out canine LILRB3 (ENSCAFG00000028453, hereinafter also indicated as "28453") gene, genome editing was performed in the same manner as in Example 2 and using the LILRB3 guide sequence (target sequence) shown in SEQ ID NO: 10.

### 2. Experiment results

By knocking out LILRB3 (ENSCAFG00000028453, hereinafter also indicated as "28453"), the elimination efficiency of abnormal cells (LILRB3 gene-deficient RasV12-expressing MDCK cells, 28453-KO-RasV12) by normal cells (MDCK cells) decreased statistically significantly (Fig. 4A).

### [Example 5]

### Production of extracellular domain D1-D2recombinant protein of 28453

### 1. Experiment method

### 28453 D1-D2

To produce the recombinant protein of 28453 D1-D2 (SEQ ID NO: 8), the expression plasmid pGEX-6p-1-28453 D1-D2 was prepared by inserting the DNA fragment encoding 28453 D1-D2 (SEQ ID NO: 9) into the EcoRI/XhoI restriction enzyme site (Fig. 4B).

IPTG (final concentration 0.1 mM) was added to the culture medium of BL21 transformed with pGEX-6p-1-DLA88-alpha3, and the expression of the recombinant protein was induced at 25°C for 2 hr. The harvested Escherichia coli was solubilized, and a recombinant protein of 28453 D1-D2 (SEQ ID NO: 8) was purified from the extract by using Gluthatione Sepharose 4B.

### 2. Experiment results

The desired recombinant protein of "28453-D1/2" (SEQ ID NO: 8) was obtained.

### [Example 6]

### Evaluation of binding property of 28453-D1/2 and DLA88-α3

### 1. Experiment method

GST-28453-D1/2-HA and GST-DLA88-α3-Flag were mixed at 1:1 in PBS containing 0.1% Triton X-100 (0.1% Triton X-100-PBS). Then, the mixture was allowed to stand at 37°C for 2 hr, beads carrying an anti-Flag antibody (hereinafter to be referred to as "Flagbeads") were added, and the mixture was mixed by inverting at 4°C for 30 min. Flagbeads were washed 3 times with 0.1% Triton X-100-PBS and then developed and separated by SDS-PAGE. The immunoprecipitated GST-DLA88-α3-Flag was detected by anti-Flag antibody, and the coprecipitated GST-28453-D1/2-HA was detected by antiHA antibody.

### 2. Experiment results

28453-D1/2 labeled with HA and DLA88-α3 labeled with FLAG were mixed and immunoprecipitated, and both HA and FLAG labels were observed. The result shows that 28453-D1/2 and DLA88-α3 have binding activity (Fig. 4C).

### [Example 7]

### Evaluation of cell non-autonomous effect between normal cell and mutant cell

### 1. Experiment method

In the same manner as in Example 1 and 3 except that a recombinant protein treatment (300 nM or 1,000 nM) was performed in addition to the doxycycline treatment, a cell non-autonomous effect was evaluated.

### 2. Experiment results

When 300 nM or 1,000 nM of 28453-D1/2 was added to a mixed culture of normal cells (MDCK cells) and abnormal cells (RasV12-expressing cells), a statistically significant decrease in the elimination rate of mutant cells was found in a 1,000 nM addition group (Fig. 4D).

### [Example 8]

### Chemical reaction theory analysis of the binding between recombinant protein containing only α3 domain of DLA88 and recombinant protein of the D1/D2 domains of LILRB3

### 1. Experiment method

BLItz^{™} (PRIMETECH) was used to analyze the dynamics between DLA88-α3 and LILRB3-D1/D2. A GST sensor and an anti-GST antibody-immobilized sensor (PRIMETECH) were used. GST-DLA88-α3 and GST-LILRB3-D1/D2 were separately incubated at 37°C for 2 hr. GST-DLA88-α3 was immobilized on the GST sensor as a ligand molecule for 5 min and completely covered with the GST sensor. After washing with PBS-T (5% Tween-PBS) for 30 sec, GST-LILRB3 D1/D2 was loaded as a sample while trembling for 2 min. In addition to association kinetics, dissociation kinetics was evaluated in PBS-T buffer for 2 min. The recombinant protein GST-GFP was used as a control.

### 2. Experiment results

As a result of the above-mentioned measurement, the association constant Kd was determined to be 6.54±0.28 µM.

### [Example 9]

### Cell competition experiment in human cells

### 1. Experiment method

In the previous Examples, MDCK cells of a cell line derived from canine renal tubular epithelial cell were used as a model experimental system for the competitive phenomenon of epithelial cells. In this Example, considering the application to humans, HaCaT cells of a cell line of human epidermal keratinocyte were used.

In the same manner as in the Example of MDCK cells, RasV12-expressing HaCaT cells (LILRB3^{WT}-RasV12) expressing GFP-RasV12 were produced by gene transfer. Normal HaCaT cells and RasV12-expressing HaCaT cells (LILRB3^{WT}-RasV12) were mixed at a ratio of 30:1, and the efficiency of elimination of the RasV12-expressing HaCaT cells as abnormal cells to the apical side by cell competition was measured as the ratio of the number of cells that escaped to the total number of RasV12-expressing cells, every 2 hr starting from the addition of doxycycline 20 hr after seeding.

### 2. Experiment results

The results are shown in Fig. 6. When doxycycline and recombinant protein 28453-D1/2 were added for treatment at the same time (LILRB3^{WT} + 28453-D1/2), the elimination efficiency of RasV12-expressing HaCat cells by normal HaCat cells increased monotonically after the addition of doxycycline and reached 40% 44 hr after seeding. In contrast, when doxycycline alone was added (LILRB3^{WT}), the elimination efficiency of RasV12-expressing HaCat cells by normal HaCat cells increased only slightly. From this result, it was shown that the recombinant protein of the D1/D2 domains of canine LILRB3 promotes cell competition in human HaCat cells. In LILRB3-KO cells, the elimination efficiency was not rescued by α3 treatment.

### [Example 10]

### Tumor formation of human HaCat cells and RasV12-expressing HaCat cells transplanted into nude mouse

### 1. Experiment method

Human HaCat normal cells (HaCat):RasV12-expressing human HaCat cells (RasV12) were mixed with Matrigel under the condition of 3:1, 10:1 or 30:1 in the presence or absence of DLA88-α3. Pellets of the mixed cells were transplanted into the abdomen of nude mice, and the total volume and weight of the tumor mass formed in 2 weeks were measured.

### 2. Experiment results

The results are shown in Fig. 7. Increase in the normal cell rate suppressed tumor growth, indicating that normal cell plays an inhibitory role in tumor formation. In addition, simultaneous injection of α3 recombinant protein substantially suppressed tumor formation specifically under mixing conditions. The suppressive effect of α3 was not observed at a mixing ratio of 30:1, but was most clearly observed at a mixing ratio of 10:1. At a ratio of 10:1, RasV12 cells mainly occupied the tumor area of the frozen section, and normal cells dominantly occupied the mosaic-like region tracing α3. The inhibitory effect of α3 on HLA-tKO was further tested. The tumor size of RasV12 HLA6tKO cells was slightly smaller than that of RasV12 WT cells, but RasV12 cells dominantly occupied the tumor region. The α3 treatment showed statistically significant suppression. These results suggest that LILRB3 surrounding normal cells is stimulated by α3 and promotes eliminating force.

### Induction of polar migration of adjacent normal cells surrounding normal cells by HLA-B/LILRB3 interaction 1. Experiment method

### Live imaging and particle image velocity measurement

On a collagen-coated bottom dish, normal HaCaT and HaCaT-RasV12 cells were mixed at a ratio of 30:1 and cultured at 37°C for 16 hr until the cells formed a single layer. The cells were incubated in the presence of doxycycline. 8 Hours after incubation, live imaging movies were acquired every 48 hr and 5 min using the JuLI^{™} stage (NanoEntek). In the case of particle image velocimetry (PIV) analysis, the video per 2 hr was analyzed by PIV of ImageJ plug-in. The PIV parameter set was as follows: NMT normalized median test parameter noise (0.3) and NMT threshold (0.8), DMT dynamics mean test parameter C1 (0.5), parameter C2 (0.3).

### evaluation of Caspase-3 activity

Normal HaCaT cells and HaCaT-RasV12 cells were mixed at a ratio of 50:1 and seeded on a collagen gel. After forming a single phase, tetracycline and α3 were added. After 16 hr, the cells were immobilized, and the antibody that recognizes cleaved Caspase-3 and Actin were stained with Pallodin. The samples were observed with a confocal microscope. The XZ image at that time is shown. (-): non-treatment, (+): α3 treatment.

### 2. Experiment results

The results are shown in Fig. 8. Since RasV12 cells are mostly removed between 16 hr and 36 hr, images of 16 hr to 36 hr were subjected to PIV analysis. Interestingly, some of the normal cells migrated towards RasV12 cells at 28 hr and before apical extrusion at 32 hr, and 4 hr later, RasV12 cells are extruded from the epithelial monolayer. This polar migration of normal cells near RasV12 cells increased by α3 treatment, but the number of polar normal cells almost disappeared by LILRB3 knockout (left figure). These results suggest that the interaction between MHC-I and LILRB3 induces polar migration of adjacent normal cells and eliminates RasV12 cells. In addition, it was demonstrated that apoptosis was induced in the cells eliminated by the addition of the α3 domain (right figure).

Apoptosis of mutant cells surrounded by normal cells when α 3 was treated was detected with an antibody capable of detecting activation-dependent cleavage of Caspase-3. The α3 treatment increased the cells eliminated during co-culture, and the cleaved caspase-3 positive cells in the eliminated cells increased (Fig. 8, right figure).

### [Example 11]

Human papillomavirus (HPV) is known to cause canceration by degrading the tumor suppressor gene Scribble after infecting cells. On the other hand, it is known that cells that suppress the expression of Scribble are eliminated while inducing apoptosis by peripheral normal cells (Fig. 9, left figure).

### 1. Experiment method

### Elimination of Scribble knockdown cells via α3 (ECA-1-ex3) ECAR

Elimination of Scribble knockdown cells was promoted via α 3 (ECA-1-ex3) ECAR. Tetracycline-inducible Scribble knockdown MDCK cells (GFP-positive cells) were mixed and seeded together with normal cells or 28453 (ECAR) at a ratio of 1:10 (MDCK:SCRB^{KD} = 10:1). After forming a single phase, tetracycline and α3 were added. The ratio of GFP-positive cells (Scribble knockdown cells) to the number of cells in the microscope field 24 hr later was calculated. ns.: not significant, **P< 0.01.

### 2. Experiment results

The results are shown in Fig. 9. The SCRB^{KD} cells had a residual rate of about 35%, but treatment with α3 resulted in a residual rate of about 20%. This is because the effect of α3 promoted the elimination of SCRBKD cells. In ECAR knockout cells, the residual rate increased irrespective of the presence or absence of α3. These results suggest that α3 promotes the elimination of SCRB^{KD} cells via ECAR.

### [Example 12]

### 1. Experiment method

Elimination of a HPV whole genome stable cell (MCF10A cell) is promoted by α3 treatment. After making MCF10A (HPV18) cells that stably retain the entire HPV genome (HPV18 genome) identifiable by CMFDA (green fluorescence staining) after mixing and culturing, they were mixed with normal MCF10A at a ratio of 10:1 (Normal MCF10A:HPV18 = 10:1) and seeded. The ratio of GFP-positive cells (HPV18 cells) to the number of cells in the microscope field 24 hr later was calculated. Cont: residual ratio of HPV18 in epithelial cell layer, a3: residual ratio of HPV18 cells after α3 treatment. ns.: not significant, **P< 0.01.

### 2. Experiment results

The results are shown in Fig. 10. The residual rate of the cells (HPV18) that stably retain the entire HPV genome decreased by the treatment with α3. This means that α3 also promotes elimination of cells with the entire HPV genome.

### [Example 13]

### Identification of novel cell membrane protein as cell competition regulating factor

### 1. Experiment method

### Verification of induction level of LILRB3 and cell removal efficiency

LILRB3 is induced specifically upon co-culture. Normal cells and RasV12 cells were co-cultured at a ratio of 1:1 on a silicone plate having a hardness of 0.5 kPa. After forming a single phase, the cells were treated with tetracycline for 8 hr. The cells were collected, the induction level of LILRB3 was measured by quantitative PCR, and shown as a graph. *P < 0.05, **P < 0.01, ****P < 0.001 by Student's t-test (Fig. 11 Upper left figure).

LILR3 is induced on the normal cell side at the protein level under co-culture conditions. After seeding normal cells only, RasV12 cells only, and a mixture of normal-mutant cells on a collagen gel, tetracycline was added when a single phase was formed. Then, for 24 hr, the cells were immobilized and stained without a membrane permeation treatment. Anti-LILRB3 antibody staining, HLA-B. Representative XY image (Fig. 11, left figure) Quantification of protein induction level of LILRB3 (Fig. 11, Lower left figure). Scale bars: 10 µm. *P < 0.05, **P < 0.01, ****P < 0.001 by Student's t-test.

LILRB3 deficiency in MDCK suppresses the elimination of mutant cells. RasV12 cells of MDCK were each mixed with MDCK-WT, -AltR-KO or -AltR-KO-OE cells, allowed to form a single phase on collagen, and tetracycline was added. Then, the cell elimination efficiency in 24 hr was calculated. The cells were stained with Phalloidin, Hoechst. Representative XZ image (Fig. 12, Upper left figure) and the results of quantification of elimination efficiency (Fig. 12, Lower left figure) are shown. Scale bars: 10 µm. *P < 0.05, **P < 0.01, ****P < 0.001 by Student's t-test.

Knockout of LILRB3 in normal HaCaT cells suppresses the elimination of mutant cells. RasV12 HaCaT cells were mixed with normal HaCaT-WT, -LILRB3-KO (LILRB3^{KO}) or -LILRB5-KO (LILRB5^{KO}) cells, allowed to form a single phase on collagen, and tetracycline was added. The cells were then immobilized for 16 hr and stained with Phalloidin and Hoechst Representative XZ image is shown (Fig. 12, Upper right figure). In addition, quantitative data on elimination efficiency is shown as a graph (Fig. 12, Lower right figure). Scale bars: 10 µm. *P < 0.05, **P < 0.01, ****P < 0.001 by Student's t-test.

### 2. Experiment results

The results are shown in Fig. 11 and Fig. 12. Non-cell autonomous induction of LILRB3 mRNA under mixing conditions and the most homologous LILRB3 protein level in normal cells surrounding RasV12 cells were confirmed. LILRB3-KO in normal cells suppressed apical extrusion.

This application is based on a patent application No. 2019-063594 filed in Japan (filing date: March 28, 2019) and a patent application No. 2019-069777 filed in Japan (filing date: April 1, 2019), the contents of which are incorporated in full herein.

### [Industrial Applicability]

The present invention can be utilized as a medicament for the prophylaxis or treatment of cancer and virus infection. In addition, the present invention can also be utilized as a medicament for improving an engraftment failure in organ transplantation, tissue transplantation, or cell transplantation, or suppressing progression of the symptoms of neurodegenerative diseases.

## Claims

1. A cell competition regulator comprising a protein containing α3 domain of MHC class Ia or an agonist antibody against LILRB3, or a substance that inhibits binding between MHC class Ia and LILRB3.

2. The regulator according to claim 1, wherein the regulator comprises the protein containing α3 domain of MHC class Ia or the agonist antibody against LILRB3, and the cell competition is regulated by promoting cell competition.

3. The regulator according to claim 2, wherein the MHC class Ia is HLA-B or DLA-88.

4. The regulator according to claim 2 or 3, wherein the protein containing α3 domain of MHC class Ia comprises at least one protein selected from the group consisting of the following:
a) a protein having the amino acid sequence shown in SEQ ID NO: 1,
b) a protein having an amino acid sequence resulting from deletion, substitution, or addition of one to several amino acids in the amino acid sequence shown in SEQ ID NO: 1, and specifically binding to a protein containing D1 and D2 domains of LILRB3,
c) a protein having an amino acid sequence having at least 90% similarity to the amino acid sequence shown in SEQ ID NO: 1, and specifically binding to a protein containing D1 and D2 domains of LILRB3, and
d) a protein containing α3 domain of an orthologue of DLA-88.

5. The regulator according to claim 4, wherein the protein containing the D1 and D2 domains of LILRB3 is a protein having the amino acid sequence shown in SEQ ID NO: 8.

6. The regulator according to any one of claims 2 to 5, wherein the cell competition is regulated by eliminating abnormal cells by a normal cell not belonging to an immune system.

7. The regulator according to claim 6, wherein the abnormal cell is a mutant cell, a cancer cell, or a virus-infected cell.

8. The regulator according to claim 7, wherein the regulator is a prophylactic or therapeutic agent for cancer or a virus infectious disease.

9. The regulator according to claim 1, wherein the regulator comprises the substance that inhibits binding between MHC class Ia and LILRB3 and the cell competition is regulated by suppressing cell competition.

10. The regulator according to claim 9, wherein the substance that inhibits binding between MHC class Ia and LILRB3 is at least one kind of substance selected from the group consisting of a protein containing D1 and D2 domains of LILRB3, an antibody against D1 and D2 domains of LILRB3, an aptamer, and an inhibitor of LILRB3 expression.

11. The regulator according to claim 10, wherein the protein containing D1 and D2 domains of LILRB3 comprises at least one protein selected from the following:
e) a protein having the amino acid sequence shown in SEQ ID NO: 8,
f) a protein having an amino acid sequence resulting from deletion, substitution, or addition of one to several amino acids in the amino acid sequence shown in SEQ ID NO: 8, and specifically binding to a protein containing α3 domain of MHC class Ia,
g) a protein having an amino acid sequence having at least 80% similarity to the amino acid sequence shown in SEQ ID NO: 8, and specifically binding to a protein containing α3 domain of MHC class Ia, and
h) a protein containing D1 and D2 domains of an orthologue of ENSCAFG00000028453.

12. The regulator according to any one of claims 9 to 11, wherein the cell competition is regulated by eliminating transplanted cells by a normal cell not belonging to an immune system.

13. The regulator according to any one of claims 9 to 12, wherein the cell competition is suppressed by improving an engraftment failure in organ transplantation, tissue transplantation, or cell transplantation, or suppressing progression of symptoms of a neurodegenerative disease, or a muscular degenerative disease.

14. The regulator according to any one of claims 1 to 13, which is used in combination with other medicament and/or other treatment method.


SEQUENCE LISTING

<110>  Waseda University

<120>  Agent for regulation of cell competition

<130>  093020

<150>  JP 2019-063594
<151>  2019-03-28

<150>  JP 2019-069777
<151>  2019-04-01

<160>  16

<170>  PatentIn version 3.5

<210>  1
<211>  93
<212>  PRT
<213>  Canis familiaris

<220>
<221>  MISC_FEATURE
<223>  DLA88 alpha3

<400>  1

Ala Glu Pro Pro Ser Thr Arg Val Thr Arg His Pro Ile Ser Asp His
1               5                   10                  15

Glu Val Thr Leu Arg Cys Trp Ala Leu Gly Phe Tyr Pro Ala Glu Ile
            20                  25                  30

Thr Leu Thr Trp Gln Arg Asp Gly Glu Asp Gln Thr Gln Asp Thr Glu
        35                  40                  45

Val Val Asp Thr Arg Pro Ala Gly Asp Gly Thr Phe Gln Lys Trp Ala
    50                  55                  60

Ala Val Val Val Pro Ser Gly Gln Glu Gln Arg Tyr Thr Cys His Val
65                  70                  75                  80

Gln His Glu Gly Leu Ala Glu Pro Val Thr Arg Arg Trp
                85                  90

<210>  2
<211>  279
<212>  DNA
<213>  Canis familiaris

<220>
<221>  misc_feature
<223>  DLA88 alpha3

<400>  2
gcagaacccc ccagcacacg tgtgacccgc caccccatct ctgaccatga ggtcaccctg      60

aggtgctggg cgctgggctt ctaccctgcg gagatcaccc tgacctggca gcgggatggg     120

gaggaccaga cccaggacac agaggttgtg gacacaaggc ctgcaggaga tgggaccttc     180

cagaagtggg cggccgtggt ggtgccctct ggacaggagc agagatacac gtgccacgtg     240

cagcatgagg ggctggcgga gcctgtcacg cggagatgg                            279


<210>  3
<211>  19
<212>  DNA
<213>  Artificial Sequence

<220>
<223>  TAP1-KO

<400>  3
tgctccgtgc tggtcccca                                                   19


<210>  4
<211>  19
<212>  DNA
<213>  Artificial Sequence

<220>
<223>  DLA88-KO

<400>  4
tccactcggt acagctgtg                                                   19


<210>  5
<211>  19
<212>  DNA
<213>  Artificial Sequence

<220>
<223>  DLA79-KO

<400>  5
cactgggcag tcgcctacg                                                   19


<210>  6
<211>  19
<212>  DNA
<213>  Artificial Sequence

<220>
<223>  DLA64-KO

<400>  6
gacgcggcga gtccgaagg                                                   19


<210>  7
<211>  19

<212>  DNA
<213>  Artificial Sequence

<220>
<223>  DLA12-KO

<400>  7
gccgtgaccc tgacccggg                                                    19


<210>  8
<211>  200
<212>  PRT
<213>  Canis familiaris


<220>
<221>  MISC_FEATURE
<223>  28453-D1/2

<400>  8

Thr Ile Trp Ala Glu Pro Gly Ser Val Ile Pro Met Glu Thr Ser Val
1               5                   10                  15


Thr Leu Trp Cys Lys Gly Ser Leu Lys Ala Gln Lys Tyr Val Leu Tyr
            20                  25                  30


Lys Asp Gly Tyr Pro Glu Thr Trp Glu Glu Gln Lys Pro Leu Glu Pro
        35                  40                  45


Lys Asp Lys Ala Lys Phe Pro Ile Thr Asp Met Thr Ser Val Tyr Ala
    50                  55                  60


Gly Arg Tyr His Cys Tyr Tyr Glu Ser Pro Thr Gly Trp Ser Glu Pro
65                  70                  75                  80


Ser Asp Gln Leu Glu Leu Val Val Thr Gly Met His Ser Lys Pro Ser
                85                  90                  95


Leu Ser Val Leu Pro Ser Pro Ile Val Pro Ser Gly Gly Asn Val Thr
            100                 105                 110


Leu Gln Cys Gly Ser Trp Arg Gly Phe Asn Arg Phe Ile Leu Met Arg
        115                 120                 125


Glu Val Glu Gly Gln Pro Ser Trp Thr Arg Asp Ser Gln Arg Ala Pro
    130                 135                 140


Ser Gly Glu Phe Gln Ala Leu Phe Pro Val Gly Pro Val Ile Pro Ser
145                 150                 155                 160


Leu Met Trp Thr Phe Arg Cys His Gly Phe Tyr Ser Asn Thr Pro Gln

165                 170                 175

Val Trp Ser Leu Pro Ser Asp Pro Leu Glu Leu Leu Val Ser Gly Val
        180                 185                 190

Leu Gly Lys Pro Ser Leu Leu Thr
        195                 200

<210>  9
<211>  600
<212>  DNA
<213>  Canis familiaris

<220>
<221>  misc_feature
<223>  28453-D1/2

<400>  9
accatctggg ctgagccagg ctctgtgatc cccatggaga catcagtgac cctctggtgt      60

aaggggagcc tgaaagccca gaagtacgtc ctgtataaag atggataccc agagacctgg     120

gaggagcaga agccactgga gccgaaggac aaggccaagt tccccatcac agacatgaca     180

agtgtatatg caggacggta tcactgttac tatgaaagcc ccactggctg gtcggagccc     240

agtgaccagc tggagctggt ggtgacaggg atgcacagca aacccagcct ctcagttctg     300

cccagcccta tcgtgccctc aggagggaac gtgaccctcc agtgtggttc atggagagga     360

tttaacaggt ttattctgat gagggaagta gaaggccagc cctcctggac ccgggactcc     420

cagcgagccc ccagtggaga gttccaggcc ctgttccctg tgggccccgt gatccccagc     480

ctcatgtgga cgttcagatg ccatggtttt tacagcaaca ccccccaggt gtggtcactc     540

cccagtgacc ctctggagct gctggtctca ggtgtgttgg ggaagccctc cctcttgacc     600

<210>  10
<211>  19
<212>  DNA
<213>  Artificial Sequence

<220>
<223>  LILRB3-KO

<400>  10
taccgagctg ggaagtacg                                                   19

<210>  11
<211>  92
<212>  PRT
<213>  Homo sapiens

<220>
<221>  MISC_FEATURE

<223>  HLA-B alpha3

<400>  11

Asp Pro Pro Lys Thr His Val Thr His His Pro Ile Ser Asp His Glu
1               5                   10                  15

Ala Thr Leu Arg Cys Trp Ala Leu Gly Phe Tyr Pro Ala Glu Ile Thr
            20                  25                  30

Leu Thr Trp Gln Arg Asp Gly Glu Asp Gln Thr Gln Asp Thr Glu Leu
        35                  40                  45

Val Glu Thr Arg Pro Ala Gly Asp Arg Thr Phe Gln Lys Trp Ala Ala
    50                  55                  60

Val Val Val Pro Ser Gly Glu Glu Gln Arg Tyr Thr Cys His Val Gln
65                  70                  75                  80

His Glu Gly Leu Pro Lys Pro Leu Thr Leu Arg Trp
                85                  90

<210>  12
<211>  188
<212>  PRT
<213>  Homo sapiens

<220>
<221>  MISC_FEATURE
<223>  LILRB3-D1/2

<400>  12

Gly Ser Pro Val Thr Ile Trp Cys Gln Gly Ser Gln Glu Ala Gln Glu
1               5                   10                  15

Tyr Arg Leu His Lys Glu Gly Ser Pro Glu Pro Leu Asp Arg Asn Asn
            20                  25                  30

Pro Leu Glu Pro Lys Asn Lys Ala Arg Phe Ser Ile Pro Ser Met Thr
        35                  40                  45

Glu His His Ala Gly Arg Tyr Arg Cys His Tyr Tyr Ser Ser Ala Gly
    50                  55                  60

Trp Ser Glu Pro Ser Asp Pro Leu Glu Met Val Met Thr Gly Ala Tyr
65                  70                  75                  80

Ser Lys Pro Thr Leu Ser Ala Leu Pro Ser Pro Val Val Ala Ser Gly
                85                  90                  95

Gly Asn Met Thr Leu Arg Cys Gly Ser Gln Lys Gly Tyr His His Phe
            100                 105                 110

Val Leu Met Lys Glu Gly Glu His Gln Leu Pro Arg Thr Leu Asp Ser
        115                 120                 125

Gln Gln Leu His Ser Arg Gly Phe Gln Ala Leu Phe Pro Val Gly Pro
    130                 135                 140

Val Thr Pro Ser His Arg Trp Arg Phe Thr Cys Tyr Tyr Tyr Tyr Thr
145                 150                 155                 160

Asn Thr Pro Trp Val Trp Ser His Pro Ser Asp Pro Leu Glu Ile Leu
                165                 170                 175

Pro Ser Gly Val Ser Arg Lys Pro Ser Leu Leu Thr
            180                 185

<210>  13
<211>  278
<212>  PRT
<213>  Canis familiaris

<220>
<221>  MISC_FEATURE
<223>  DLA88 full-length

<400>  13

Met Gly Ser His Ser Leu Arg Tyr Phe Tyr Thr Ser Val Ser Arg Pro
1               5                   10                  15

Gly Arg Gly Asp Pro Arg Phe Ile Ala Val Gly Tyr Val Asp Asp Thr
            20                  25                  30

Gln Phe Val Arg Phe Asp Ser Asp Ala Ala Thr Gly Arg Thr Glu Pro
        35                  40                  45

Arg Ala Pro Trp Val Glu Gln Glu Gly Pro Glu Tyr Trp Asp Gly Glu
    50                  55                  60

Thr Arg Lys Val Lys Glu Thr Ala Gln Val Tyr Arg Val Asp Leu Asp
65                  70                  75                  80

Thr Leu Arg Gly Tyr Tyr Asn Gln Ser Glu Ala Gly Ser His Thr Ile
                85                  90                  95

Gln Thr Met Tyr Gly Cys Asp Leu Gly Pro Gly Gly Arg Leu Leu Arg
            100                 105                 110

Gly Tyr Arg Gln Asp Ala Tyr Asp Gly Ala Asp Tyr Ile Ala Leu Asn
        115                 120                 125

Glu Asp Leu Arg Ser Trp Thr Ala Ala Asp Thr Ala Ala Gln Ile Thr
    130                 135                 140

Arg Arg Lys Trp Glu Ala Ala Gly Val Ala Glu Leu Gln Trp Arg Asn
145                 150                 155                 160

Tyr Leu Glu Thr Thr Cys Val Glu Trp Leu Gln Arg Tyr Leu Glu Met
                165                 170                 175

Gly Lys Glu Thr Leu Leu Arg Ala Glu Pro Pro Ser Thr Arg Val Thr
            180                 185                 190

Arg His Pro Ile Ser Asp His Glu Val Thr Leu Arg Cys Trp Ala Leu
        195                 200                 205

Gly Phe Tyr Pro Ala Glu Ile Thr Leu Thr Trp Gln Arg Asp Gly Glu
    210                 215                 220

Asp Gln Thr Gln Asp Thr Glu Val Val Asp Thr Arg Pro Ala Gly Asp
225                 230                 235                 240

Gly Thr Phe Gln Lys Trp Ala Ala Val Val Val Pro Ser Gly Gln Glu
                245                 250                 255

Gln Arg Tyr Thr Cys His Val Gln His Glu Gly Leu Val Glu Pro Val
            260                 265                 270

Thr Arg Arg Trp Glu Pro
        275

<210>  14
<211>  588
<212>  PRT
<213>  Canis familiaris

<220>
<221>  MISC_FEATURE
<223>  28453 full-length

<400>  14

Met Thr Pro Ile Leu Thr Ala Leu Leu Cys Leu Gly Leu Ser Val Gly
1               5                   10                  15

Ser Arg Asp Ser Val Gln Ala Gly Thr Leu Pro Lys Pro Thr Ile Trp

            20                  25                  30

Ala Glu Pro Gly Ser Val Ile Pro Met Glu Thr Ser Val Thr Leu Trp
        35                  40                  45

Cys Lys Gly Ser Leu Lys Ala Gln Lys Tyr Val Leu Tyr Lys Asp Gly
    50                  55                  60

Tyr Pro Glu Thr Trp Glu Glu Gln Lys Pro Leu Glu Pro Lys Asp Lys
65                  70                  75                  80

Ala Lys Phe Pro Ile Thr Asp Met Thr Ser Val Tyr Ala Gly Arg Tyr
                85                  90                  95

His Cys Tyr Tyr Glu Ser Pro Thr Gly Trp Ser Glu Pro Ser Asp Gln
            100                 105                 110

Leu Glu Leu Val Val Thr Gly Met His Ser Lys Pro Ser Leu Ser Val
        115                 120                 125

Leu Pro Ser Pro Ile Val Pro Ser Gly Gly Asn Val Thr Leu Gln Cys
    130                 135                 140

Gly Ser Trp Arg Gly Phe Asn Arg Phe Ile Leu Met Arg Glu Val Glu
145                 150                 155                 160

Gly Gln Pro Ser Trp Thr Arg Asp Ser Gln Arg Ala Pro Ser Gly Glu
                165                 170                 175

Phe Gln Ala Leu Phe Pro Val Gly Pro Val Ile Pro Ser Leu Met Trp
            180                 185                 190

Thr Phe Arg Cys His Gly Phe Tyr Ser Asn Thr Pro Gln Val Trp Ser
        195                 200                 205

Leu Pro Ser Asp Pro Leu Glu Leu Leu Val Ser Gly Val Leu Gly Lys
    210                 215                 220

Pro Ser Leu Leu Thr Gln Gln Gly Pro Val Val Thr Ser Gly Gln Asn
225                 230                 235                 240

Leu Thr Leu Gln Cys Leu Ser Asp Val Ser Tyr Asp Arg Phe Ala Leu
                245                 250                 255

Phe Lys Glu Gly Thr Ser Asp Leu Leu Gln Leu His Gly Arg Gln Thr
            260                 265                 270

Gln Ala Gly Phe Ser Gly Ala Asp Phe Ser Leu Ser Pro Val Lys Ser
        275                 280                 285

Ser Arg Gly Gly Gln Tyr Thr Cys Tyr Gly Gly His Asn Leu Ser Ser
    290                 295                 300

Gln Trp Ser Ala Pro Ser Asp Pro Leu Asp Ile Leu Val Thr Gly Gln
305                 310                 315                 320

Leu Ser Tyr Thr Pro Ser Leu Arg Val His Pro Gly Pro Thr Val Ala
                325                 330                 335

Pro Gly Glu Asn Val Thr Leu Leu Cys Gln Ser Leu Ser Asn Val Asp
            340                 345                 350

Thr Phe Leu Leu Ser Lys Asp Gly Ala Ala Asp Ala Ser Leu Arg Leu
        355                 360                 365

Arg Ser Lys Tyr Arg Ala Gly Lys Tyr Glu Ala Glu Phe Ser Met Ser
    370                 375                 380

Pro Val Thr Pro Ala His Gly Gly Thr Tyr Arg Cys Tyr Gly Ser Ile
385                 390                 395                 400

Lys Thr Ser Pro Tyr Leu Leu Ser Tyr Pro Ser Val Pro Val Glu Ile
                405                 410                 415

Leu Val Ser Gly Ser Phe Gly His His Gly Pro Pro Pro Thr Met Ser
            420                 425                 430

Ser Pro Thr Ala Ala Pro Ala Gln Gly Pro Ser Trp Tyr Leu Tyr Val
        435                 440                 445

Leu Ile Gly Ala Ser Val Ala Phe Ile Leu Met Leu Cys Leu Leu Ile
    450                 455                 460

Leu Leu Leu Val Arg Gln Gln Arg Arg Gly Lys Cys Arg Lys Ser Thr
465                 470                 475                 480

Gly Asp Ala Asp Pro Glu Pro Lys Asp Arg Gly Leu Gln Asp Ser Ser
                485                 490                 495

Arg Pro Ala Ala Val Ala Gln Glu Glu Thr Leu Tyr Ala Ala Val Gln
            500                 505                 510

Asp Ser Trp Pro Glu Asp Gly Val Lys Leu Asp His Trp Gln Asn Thr
        515                 520                 525

Gln Asp Glu Asp Pro Gln Val Val Thr Tyr Ala Gln Ala Thr Ala Ser
    530                 535                 540

Asp Ala Pro Pro Asp Val Thr Tyr Ala Gln Leu Asn His Leu Thr Leu
545                 550                 555                 560

Arg Gln Lys Thr Ser Ala Ser Ser Pro Ser Gln Ser Glu Glu Pro Pro
                565                 570                 575

Ala Glu Pro Ser Val Tyr Ala Ala Leu Ala Ile His
            580                 585

<210>  15
<211>  362
<212>  PRT
<213>  Homo sapiens

<220>
<221>  MISC_FEATURE
<223>  HLA-B full-length

<400>  15

Met Leu Val Met Ala Pro Arg Thr Val Leu Leu Leu Leu Ser Ala Ala
1               5                   10                  15

Leu Ala Leu Thr Glu Thr Trp Ala Gly Ser His Ser Met Arg Tyr Phe
            20                  25                  30

Tyr Thr Ser Val Ser Arg Pro Gly Arg Gly Glu Pro Arg Phe Ile Ser
        35                  40                  45

Val Gly Tyr Val Asp Asp Thr Gln Phe Val Arg Phe Asp Ser Asp Ala
    50                  55                  60

Ala Ser Pro Arg Glu Glu Pro Arg Ala Pro Trp Ile Glu Gln Glu Gly
65                  70                  75                  80

Pro Glu Tyr Trp Asp Arg Asn Thr Gln Ile Tyr Lys Ala Gln Ala Gln
                85                  90                  95

Thr Asp Arg Glu Ser Leu Arg Asn Leu Arg Gly Tyr Tyr Asn Gln Ser
            100                 105                 110

Glu Ala Gly Ser His Thr Leu Gln Ser Met Tyr Gly Cys Asp Val Gly
        115                 120                 125

Pro Asp Gly Arg Leu Leu Arg Gly His Asp Gln Tyr Ala Tyr Asp Gly
    130                 135                 140

Lys Asp Tyr Ile Ala Leu Asn Glu Asp Leu Arg Ser Trp Thr Ala Ala
145                 150                 155                 160

Asp Thr Ala Ala Gln Ile Thr Gln Arg Lys Trp Glu Ala Ala Arg Glu
                165                 170                 175

Ala Glu Gln Arg Arg Ala Tyr Leu Glu Gly Glu Cys Val Glu Trp Leu
            180                 185                 190

Arg Arg Tyr Leu Glu Asn Gly Lys Asp Lys Leu Glu Arg Ala Asp Pro
        195                 200                 205

Pro Lys Thr His Val Thr His His Pro Ile Ser Asp His Glu Ala Thr
    210                 215                 220

Leu Arg Cys Trp Ala Leu Gly Phe Tyr Pro Ala Glu Ile Thr Leu Thr
225                 230                 235                 240

Trp Gln Arg Asp Gly Glu Asp Gln Thr Gln Asp Thr Glu Leu Val Glu
                245                 250                 255

Thr Arg Pro Ala Gly Asp Arg Thr Phe Gln Lys Trp Ala Ala Val Val
            260                 265                 270

Val Pro Ser Gly Glu Glu Gln Arg Tyr Thr Cys His Val Gln His Glu
        275                 280                 285

Gly Leu Pro Lys Pro Leu Thr Leu Arg Trp Glu Pro Ser Ser Gln Ser
    290                 295                 300

Thr Val Pro Ile Val Gly Ile Val Ala Gly Leu Ala Val Leu Ala Val
305                 310                 315                 320

Val Val Ile Gly Ala Val Val Ala Ala Val Met Cys Arg Arg Lys Ser
                325                 330                 335

Ser Gly Gly Lys Gly Gly Ser Tyr Ser Gln Ala Ala Cys Ser Asp Ser
            340                 345                 350

Ala Gln Gly Ser Asp Val Ser Leu Thr Ala
        355                 360

<210>  16
<211>  631
<212>  PRT
<213>  Homo sapiens

<220>
<221>  MISC_FEATURE
<223>  LILRB3 full-length

<400>  16

Met Thr Pro Ala Leu Thr Ala Leu Leu Cys Leu Gly Leu Ser Leu Gly
1               5                   10                  15

Pro Arg Thr Arg Val Gln Ala Gly Pro Phe Pro Lys Pro Thr Leu Trp
            20                  25                  30

Ala Glu Pro Gly Ser Val Ile Ser Trp Gly Ser Pro Val Thr Ile Trp
        35                  40                  45

Cys Gln Gly Ser Gln Glu Ala Gln Glu Tyr Arg Leu His Lys Glu Gly
    50                  55                  60

Ser Pro Glu Pro Leu Asp Arg Asn Asn Pro Leu Glu Pro Lys Asn Lys
65                  70                  75                  80

Ala Arg Phe Ser Ile Pro Ser Met Thr Glu His His Ala Gly Arg Tyr
                85                  90                  95

Arg Cys His Tyr Tyr Ser Ser Ala Gly Trp Ser Glu Pro Ser Asp Pro
            100                 105                 110

Leu Glu Met Val Met Thr Gly Ala Tyr Ser Lys Pro Thr Leu Ser Ala
        115                 120                 125

Leu Pro Ser Pro Val Val Ala Ser Gly Gly Asn Met Thr Leu Arg Cys
    130                 135                 140

Gly Ser Gln Lys Gly Tyr His His Phe Val Leu Met Lys Glu Gly Glu
145                 150                 155                 160

His Gln Leu Pro Arg Thr Leu Asp Ser Gln Gln Leu His Ser Arg Gly
                165                 170                 175

Phe Gln Ala Leu Phe Pro Val Gly Pro Val Thr Pro Ser His Arg Trp
            180                 185                 190

Arg Phe Thr Cys Tyr Tyr Tyr Tyr Thr Asn Thr Pro Trp Val Trp Ser
        195                 200                 205

His Pro Ser Asp Pro Leu Glu Ile Leu Pro Ser Gly Val Ser Arg Lys
    210                 215                 220

Pro Ser Leu Leu Thr Leu Gln Gly Pro Val Leu Ala Pro Gly Gln Ser
225                 230                 235                 240

Leu Thr Leu Gln Cys Gly Ser Asp Val Gly Tyr Asn Arg Phe Val Leu
                245                 250                 255

Tyr Lys Glu Gly Glu Arg Asp Phe Leu Gln Arg Pro Gly Gln Gln Pro
            260                 265                 270

Gln Ala Gly Leu Ser Gln Ala Asn Phe Thr Leu Gly Pro Val Ser Pro
        275                 280                 285

Ser Asn Gly Gly Gln Tyr Arg Cys Tyr Gly Ala His Asn Leu Ser Ser
    290                 295                 300

Glu Trp Ser Ala Pro Ser Asp Pro Leu Asn Ile Leu Met Ala Gly Gln
305                 310                 315                 320

Ile Tyr Asp Thr Val Ser Leu Ser Ala Gln Pro Gly Pro Thr Val Ala
                325                 330                 335

Ser Gly Glu Asn Val Thr Leu Leu Cys Gln Ser Trp Trp Gln Phe Asp
            340                 345                 350

Thr Phe Leu Leu Thr Lys Glu Gly Ala Ala His Pro Pro Leu Arg Leu
        355                 360                 365

Arg Ser Met Tyr Gly Ala His Lys Tyr Gln Ala Glu Phe Pro Met Ser
    370                 375                 380

Pro Val Thr Ser Ala His Ala Gly Thr Tyr Arg Cys Tyr Gly Ser Tyr
385                 390                 395                 400

Ser Ser Asn Pro His Leu Leu Ser His Pro Ser Glu Pro Leu Glu Leu
                405                 410                 415

Val Val Ser Gly His Ser Gly Gly Ser Ser Leu Pro Pro Thr Gly Pro
            420                 425                 430

Pro Ser Thr Pro Gly Leu Gly Arg Tyr Leu Glu Val Leu Ile Gly Val
        435                 440                 445

Ser Val Ala Phe Val Leu Leu Leu Phe Leu Leu Leu Phe Leu Leu Leu
    450                 455                 460

Arg Arg Gln Arg His Ser Lys His Arg Thr Ser Asp Gln Arg Lys Thr
465                 470                 475                 480

Asp Phe Gln Arg Pro Ala Gly Ala Ala Glu Thr Glu Pro Lys Asp Arg
                485                 490                 495

Gly Leu Leu Arg Arg Ser Ser Pro Ala Ala Asp Val Gln Glu Glu Asn
            500                 505                 510

Leu Tyr Ala Ala Val Lys Asp Thr Gln Ser Glu Asp Arg Val Glu Leu
        515                 520                 525

Asp Ser Gln Ser Pro His Asp Glu Asp Pro Gln Ala Val Thr Tyr Ala
    530                 535                 540

Pro Val Lys His Ser Ser Pro Arg Arg Glu Met Ala Ser Pro Pro Ser
545                 550                 555                 560

Ser Leu Ser Gly Glu Phe Leu Asp Thr Lys Asp Arg Gln Val Glu Glu
                565                 570                 575

Asp Arg Gln Met Asp Thr Glu Ala Ala Ala Ser Glu Ala Ser Gln Asp
            580                 585                 590

Val Thr Tyr Ala Gln Leu His Ser Leu Thr Leu Arg Arg Lys Ala Thr
        595                 600                 605

Glu Pro Pro Pro Ser Gln Glu Gly Glu Pro Pro Ala Glu Pro Ser Ile
    610                 615                 620

Tyr Ala Thr Leu Ala Ile His
625                 630